# EUROPEAN PATENT APPLICATION

(11) **EP 4 265 599 A1**
(43) Date of publication of application: **25.10.2023**
(21) Application number: 21905833.6
(22) Date of filing: 17.12.2021
(51) Int. Cl.: C07D 239/42, C07D 403/12, A61K 31/506, A61P 29/00

(54) **PYRIMIDINE CARBOXAMIDE COMPOUND AND USE THEREOF**

(30) Priority: 17.12.2020 CN 202011499721; 13.12.2021 CN 202111521781
(71) Applicant: Shanghai Meiyue Biotech Development Co., Ltd., Shanghai 201206 (CN)
(72) Inventor: YAO, Yuanshan, Shanghai 201206 (CN); LUAN, Linbo, Shanghai 201206 (CN); CHEN, Yongkai, Shanghai 201206 (CN); WANG, Chaodong, Shanghai 201206 (CN)
(74) Representative: Haseltine Lake Kempner LLP
(86) International application number: PCT/CN2021/139172
(87) International publication number: WO 2022/127909

(57) **Abstract**

A pyrimidine carboxamide compound as shown in formula I, or a tautomer, mesomer, racemate, enantiomer and diastereomer thereof, or a mixture form thereof, or a pharmaceutically acceptable salt thereof, which can be used as a Vanin enzyme inhibitor, and can be used to prepare a medicament for treating various conditions, including Crohn's disease and ulcerative colitis.

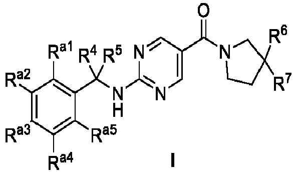

## Description

The present application claims priorities to Chinese Patent Application 2020114997213 filed on December 17, 2020 and Chinese Patent Application 2021115217815 filed on December 13, 2021. The contents of the Chinese Patent Applications are incorporated herein by reference in their entireties.

### TECHNICAL FIELD

The present disclosure relates to a pyrimidine carboxamide compound and a use thereof.

### BACKGROUND

Vanin-1 (vascular non-inflammatory molecule-1) is an exonuclease with pantetheinase activity, which mainly catalyzes the hydrolysis of pantetheine to produce pantothenic acid (VB5) and mercaptoethylamine. Coenzyme A (CoA), synthesized from VB5, regulates biotransformations such as fatty acid synthesis and oxidation as well as energy metabolism, while the reversible reaction between mercaptoethylamine and cystamine is an important sensor of oxidative stress. A growing number of studies have found that deficiency or reduced levels of mercaptoethylamine lead to enhanced γ-GCS activity, causing elevated endogenous GSH reserves in tissues, which may thereby prevent or eliminate tissue inflammation. Studies have found that mRNAof Vanin-1 is highly expressed in human colon, duodenum, endometrium, liver, kidney, gallbladder and small intestine. In patients with UC (ulcerative colitis), Vanin-1 was highly expressed and diffuse, and limited to brush borders. In addition, the expression level of Vanin-1 in the colon was still significantly higher than that of the control group during the clinically quiescent phase of UC. In the TNBS model experiment, the survival rate of Vanin-1 knockout (Vanin-1^{-/-}) mice was significantly higher than that of the model control group, and there was no significant weight loss. Moreover, 90% of Vanin-1^{-/-} mice treated with cystamine died within 5 days, indicating that cystamine completely reversed the protective effect of Vanin-1 deficiency on colitis. In addition, histopathological analysis of mice has found that the inhibition or knockout of Vanin-1 can significantly ameliorate the colon lesions in mice (Berruyer C, et al. Vanin-1-/- mice exhibit a glutathione mediated tissue resistance to oxidative stress. Mol. Cell Biol. 2004; 24: 7214-7224; Berruyer C, et al. Vanin-1 licenses inflammatory mediator production by gut epithelial cells and controls colitis by antagonizing peroxisome proliferator-activated receptor γ activity. J. Exp. Med. 2006; 203: 2817-2827).

Furthermore, Vanin-1 is also considered to play a regulatory role in cardiovascular diseases and tumor diseases. Studies have demonstrated that Vanin-1 regulates the activation of smooth muscle cells *in vitro* and the development of neointimal hyperplasia in response to carotid artery ligation *in vivo.* VNN1 gene polymorphisms are associated with blood pressure and HDL levels. In SF-1 transgenic mice, Vanin-1 deficiency prevented the mice from developing neoplasia of the adrenal cortex, suggesting a role for Vanin-1 in certain cancers. Studies in inflammatory diseases have found that Vanin-1 is highly upregulated in psoriatic skin lesions compared to normal individuals. Gene expression of VNN1 was also upregulated in whole blood from patients with childhood immune thrombocytopenia (ITP), wherein overexpression of VNN1 was associated with the progression of chronic ITP. In addition, elevated Vanin-1 has been detected in the urine of patients with a variety of renal disorders, including systemic lupus erythematosus, nephrotoxicant-induced renal injury and type 2 diabetes (Rommelaere S, et al. PPARalpha regulates the production of serum Vanin-1 by liver. FEBS Lett. 2013 Nov 15; 587(22): 3742-8).

### SUMMARY

The technical problem to be solved by the present disclosure is to overcome the lack of Vanin enzyme-based therapeutic agents in the prior art; and provided are a pyrimidine carboxamide compound and a use thereof. The pyrimidine carboxamide compound provided by the present disclosure is a compound with Vanin enzyme inhibitory activity; it has strong Vanin-1 inhibitory activity; and it can be used to treat various diseases, comprising Crohn's disease and ulcerative colitis.

The present disclosure solves the above technical problem by the following technical solutions.

The present disclosure provides a pyrimidine carboxamide compound represented by formula I, or a tautomer, mesomer, racemate, enantiomer, diastereomer thereof, or a mixture form thereof, or a pharmaceutically acceptable salt thereof; 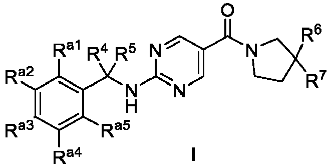
wherein, R^{a1}, R^{a3} and R^{a5} are independently H, halogen, CN, C₁-C₆ alkyl, C₁-C₆ alkyl-O-, C₁-C₆ alkyl substituted by one or more than one R^{1a}, or C₁-C₆ alkyl-O- substituted by one or more than one R^{1b};
R^{a2} and R^{a4} are independently H, halogen;
R⁴ and R⁵ are independently H, C₁-C₆ alkyl, C₁-C₆ alkyl substituted by one or more than one halogen; alternatively, R⁴ and R⁵ form 3- to 7-membered cycloalkyl together with the carbon to which they are bonded;
R⁶ and R⁷ are independently H, halogen, C₁-C₆ alkyl, C₁-C₆ alkyl substituted by one or more than one R^{1c}, -N(R^{2a}R^{2b}) or -N(R^{3a})-(C=O)-R^{3b};
alternatively, R⁶ and R⁷ form a ring B together with the carbon to which they are bonded; the ring B is 4- to 7-membered cycloalkyl, 4- to 7-membered heterocycloalkyl, 4- to 7-membered cycloalkyl substituted by one or more than one R^{1d}, or 4- to 7-membered heterocycloalkyl substituted by one or more than one R^{1e}; the heteroatom of the 4- to 7-membered heterocycloalkyl in the 4- to 7-membered heterocycloalkyl and the 4- to 7-membered heterocycloalkyl substituted by one or more than one R^{1e} is N, O or S, and the number of heteroatom is 1 or 2;
R^{1a}, R^{1b}, R^{1c}, R^{1d} and R^{1e} are independently halogen, C₁-C₄ alkyl or C₁-C₄ alkyl substituted by one or more than one halogen;
R^{2a}, R^{2b}, R^{3a} and R^{3b} are independently H or C₁-C₄ alkyl.

In some preferred embodiments of the present disclosure, some groups of the pyrimidine carboxamide compound represented by formula I, or the tautomer, mesomer, racemate, enantiomer, diastereomer thereof, or the mixture form thereof, or the pharmaceutically acceptable salt thereof are defined as follows, and unmentioned groups are the same as those described in any one of the embodiments of the present disclosure (hereinafter referred to as "in a certain embodiment").
R^{a1}, R^{a3} and R^{a5} are independently H, halogen, CN, C₁-C₆ alkyl-O-, C₁-C₆ alkyl substituted by one or more than one R^{1a}, or C₁-C₆ alkyl-O- substituted by one or more than one R^{1b};
for example, H, halogen, C₁-C₆ alkyl-O-, C₁-C₆ alkyl substituted by one or more than one R^{1a}, or C₁-C₆ alkyl-O- substituted by one or more than one R^{1b};
for another example, H, halogen or C₁-C₆ alkyl substituted by one or more than one Rla;
for still another example, halogen or C₁-C₆ alkyl substituted by one or more than one Rla;
for yet still another example, halogen.

In a certain embodiment, 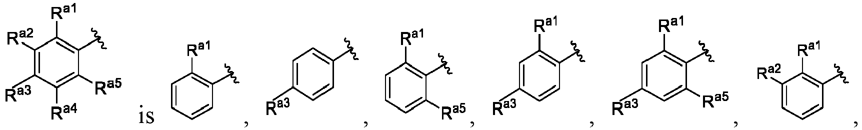 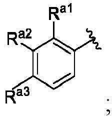 for example,

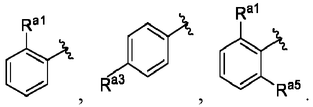

In a certain embodiment,
R^{1a} and R^{1b} are independently halogen; for example, F or Cl; for another example, F.
In a certain embodiment,
the number of R^{1a} and R^{1b} is 1, 2, 3, 4 or 5; for example, 2 or 3.

In a certain embodiment, 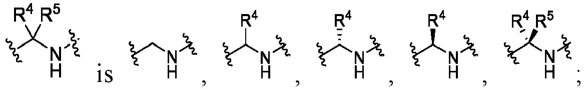
R⁴ and R⁵ are independently C₁-C₆ alkyl; alternatively, R⁴ and R⁵ form 3- to 7-membered cycloalkyl together with the carbon to which they are bonded;
for example, 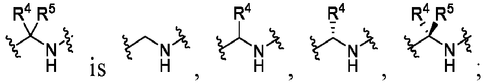
for another example, 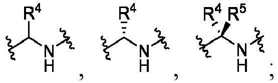
for still another example, 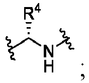 R⁴ is independently C₁-C₆ alkyl; alternatively, 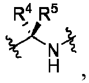 R⁴ and R⁵ form 3- to 7-membered cycloalkyl together with the carbon to which they are bonded.

In a certain embodiment,
R⁶ and R⁷ are independently H, N(R^{2a}R^{2b}) or -N(R^{3a})-(C=O)-R^{3b}; alternatively, R⁶ and R⁷ form the ring B together with the carbon to which they are bonded;
for example, R⁶ is H, and R⁷ is N(R^{2a}R^{2b}) or -N(R^{3a})-(C=O)-R^{3b}.

In a certain embodiment,
R⁶ and R⁷ form the ring B together with the carbon to which they are bonded; the ring B is 4- to 7-membered heterocycloalkyl.

In a certain embodiment,
R^{2a} and R^{2b} are independently H or C₁-C₄ alkyl; for example, C₁-C₄ alkyl.

In a certain embodiment,
R^{3a} and R^{3b} are independently H or C₁-C₄ alkyl; for example, C₁-C₄ alkyl.

In a certain embodiment,
R^{a1}, R^{a3} and R^{a5} are independently H, halogen, CN, C₁-C₆ alkyl-O-, C₁-C₆ alkyl substituted by one or more than one R^{1a}, or C₁-C₆ alkyl-O- substituted by one or more than one R^{1b};
R^{a2} and R^{a4} are independently H, halogen;
R⁴ and R⁵ are independently H, C₁-C₆ alkyl; alternatively, R⁴ and R⁵ form 3- to 7-membered cycloalkyl together with the carbon to which they are bonded;
R⁶ and R⁷ are independently H, -N(R^{2a}R^{2b}) or -N(R^{3a})-(C=O)-R^{3b};
alternatively, R⁶ and R⁷ form the ring B together with the carbon to which they are bonded; the ring B is 4- to 7-membered heterocycloalkyl;
R^{1a} is independently halogen;
R^{2a}, R^{2b}, R^{3a} and R^{3b} are independently H or C₁-C₄ alkyl.

In a certain embodiment,
R^{a1}, R^{a3} and R^{a5} are independently H, halogen, CN, C₁-C₆ alkyl-O-, C₁-C₆ alkyl substituted by one or more than one R^{1a}, or C₁-C₆ alkyl-O- substituted by one or more than one R^{1b};
R^{a2} and R^{a4} are independently H, halogen;
R⁴ and R⁵ are independently H, C₁-C₆ alkyl; alternatively, R⁴ and R⁵ form 3- to 7-membered cycloalkyl together with the carbon to which they are bonded;
R⁶ and R⁷ form the ring B together with the carbon to which they are bonded; the ring B is 4- to 7-membered heterocycloalkyl;
R^{1a} is independently halogen.

In a certain embodiment,
R^{a1}, R^{a2}, R^{a3}, R^{a4} and R^{a5} are independently H or halogen; 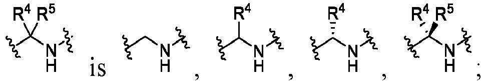 R⁴ and R⁵ are independently C₁-C₆ alkyl;
alternatively, R⁴ and R⁵ form 3- to 7-membered cycloalkyl together with the carbon to which they are bonded;
R⁶ and R⁷ form the ring B together with the carbon to which they are bonded; the ring B is 4- to 7-membered heterocycloalkyl;
for example, the ring B is tetrahydropyranyl (for example, 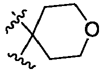 );

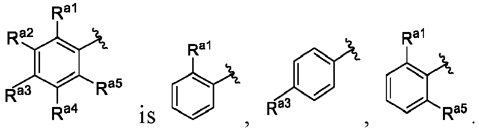

In a certain embodiment,
R^{a1}, R^{a2}, R^{a3}, R^{a4} and R^{a5} are independently H or halogen; 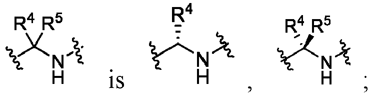
R⁴ and R⁵ are independently C₁-C₆ alkyl; alternatively, R⁴ and R⁵ form 3- to 7-membered cycloalkyl together with the carbon to which they are bonded;
R⁶ and R⁷ form the ring B together with the carbon to which they are bonded; the ring B is 4- to 7-membered heterocycloalkyl;
for example, the ring B is tetrahydropyranyl (for example, 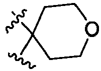 );

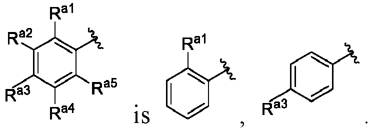

In a certain embodiment,
when R^{a1}, R^{a2}, R^{a3}, R^{a4} and R^{a5} are independently halogen, the halogen is fluorine, chlorine or bromine; for example, fluorine or chlorine; for another example, fluorine.

In a certain embodiment,
when R^{a1}, R^{a3} and R^{a5} are independently C₁-C₆ alkyl, C₁-C₆ alkyl-O-, C₁-C₆ alkyl substituted by one or more than one R^{1a}, or C₁-C₆ alkyl-O- substituted by one or more than one R^{1b}, the C₁-C₆ alkyl (such as methyl, ethyl, propyl, butyl, pentyl or hexyl) in the C₁-C₆ alkyl, C₁-C₆ alkyl-O-, C₁-C₆ alkyl substituted by one or more than one R^{1a} and C₁-C₆ alkyl-O-substituted by one or more than one R^{1b} is independently C₁-C₄ alkyl (such as methyl, ethyl, *n-*propyl, isopropyl, *n*-butyl, isobutyl, *sec*-butyl or *tert*-butyl); for example, methyl or isopropyl.

In a certain embodiment,
when R^{a1}, R^{a3} and R^{a5} are independently C₁-C₆ alkyl substituted by one or more than one R^{1a}, or C₁-C₆ alkyl-O- substituted by one or more than one R^{1b}, the number of the substituents is 1, 2, 3, 4 or 5; for example, 1, 2 or 3; for example, trifluoromethyl, trifluoromethyl-O-.

In a certain embodiment,
when R⁴ and R⁵ are independently C₁-C₆ alkyl, or C₁-C₆ alkyl substituted by one or more than one halogen, the C₁-C₆ alkyl (such as methyl, ethyl, propyl, butyl, pentyl or hexyl) in the C₁-C₆ alkyl and the C₁-C₆ alkyl substituted by one or more than one halogen is independently C₁-C₄ alkyl (such as methyl, ethyl, *n*-propyl, isopropyl, *n*-butyl, isobutyl, *sec-*butyl or *tert*-butyl); for example, methyl.

In a certain embodiment,
when R⁴ and R⁵ form 3- to 7-membered cycloalkyl together with the carbon to which they are bonded, the 3- to 7-membered cycloalkyl is independently cyclobutyl, cyclopentyl, cyclohexyl or cycloheptyl; for another example, cyclopropyl.

In a certain embodiment,
when R⁶ and R⁷ are independently halogen, the halogen is fluorine, chlorine or bromine; for example, fluorine or chlorine.

In a certain embodiment,
when R⁶ and R⁷ are independently C₁-C₆ alkyl, or C₁-C₆ alkyl substituted by one or more than one R^{1c}, the C₁-C₆ alkyl (such as methyl, ethyl, propyl, butyl, pentyl or hexyl) in the C₁-C₆ alkyl and the C₁-C₆ alkyl substituted by one or more than one R^{1c} is independently C₁-C₄ alkyl, such as methyl, ethyl, *n*-propyl, isopropyl, *n*-butyl, isobutyl, *sec*-butyl or *tert*-butyl.

In a certain embodiment,
when the ring B is 4- to 7-membered cycloalkyl, or 4- to 7-membered cycloalkyl substituted by one or more than one R^{1d}, the 4- to 7-membered cycloalkyl in the 4- to 7-membered cycloalkyl and the 4- to 7-membered cycloalkyl substituted by one or more than one R^{1d} is independently cyclobutyl, cyclopentyl, cyclohexyl or cycloheptyl.

In a certain embodiment,
when the ring B is 4- to 7-membered heterocycloalkyl, or 4- to 7-membered heterocycloalkyl substituted by one or more than one R^{1e}, the heteroatom of the 4- to 7-membered heterocycloalkyl in the 4- to 7-membered heterocycloalkyl and the 4- to 7-membered heterocycloalkyl substituted by one or more than one R^{1e} is N or O, and the number of heteroatom is 1; for example, tetrahydropyranyl; for another example,

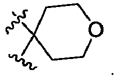

In a certain embodiment,
when R^{1a}, R^{1b}, R^{1c}, R^{1d} and R^{1e} are independently halogen, or C₁-C₄ alkyl substituted by one or more than one halogen, the halogen in the halogen and the C₁-C₄ alkyl substituted by one or more than one halogen is independently fluorine, chlorine or bromine; for example, fluorine or chlorine; for another example, fluorine.

In a certain embodiment,
when R^{1a}, R^{1b}, R^{1c}, R^{1d} and R^{1e} are independently C₁-C₄ alkyl, or C₁-C₄ alkyl substituted by one or more than one halogen, the C₁-C₄ alkyl in the C₁-C₄ alkyl and the C₁-C₄ alkyl substituted by one or more than one halogen is independently methyl, ethyl, *n*-propyl, isopropyl, *n*-butyl, isobutyl, *sec*-butyl or *tert*-butyl; for example, methyl.

In a certain embodiment,
when R^{2a}, R^{2b}, R^{3a} and R^{3b} are independently C₁-C₄ alkyl, the C₁-C₄ alkyl is independently methyl, ethyl, *n*-propyl, isopropyl, *n*-butyl, isobutyl, *sec*-butyl or *tert*-butyl; for example, methyl.

In a certain embodiment, 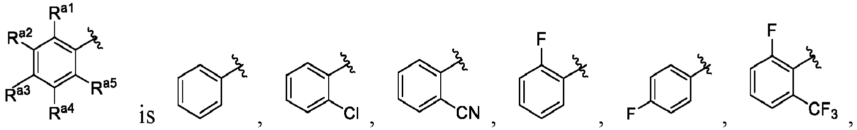 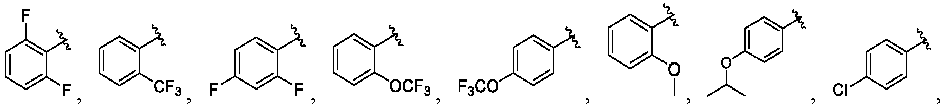 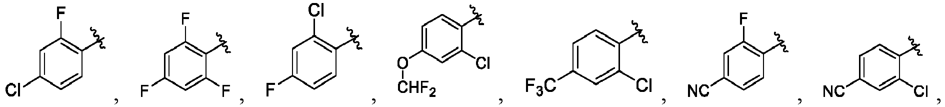 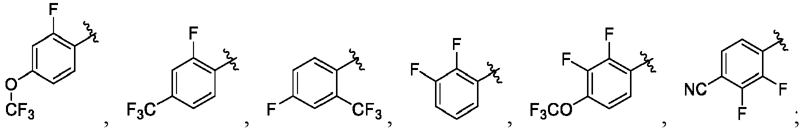 for example,

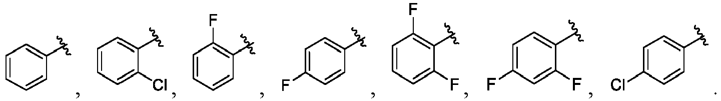

In a certain embodiment,

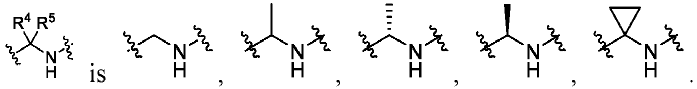

In a certain embodiment, 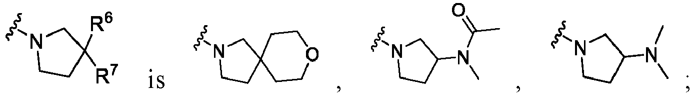 for example,

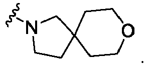

In a certain embodiment,
the pyrimidine carboxamide compound represented by formula I is selected from any one of the following structures:

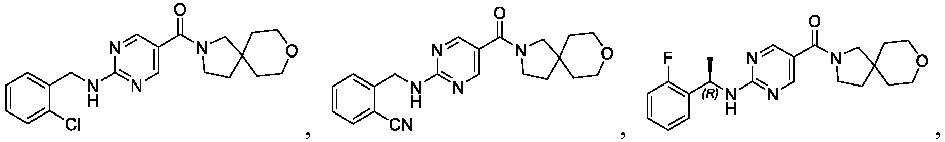

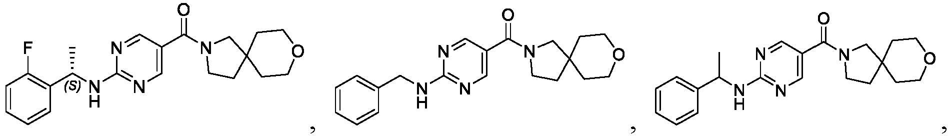

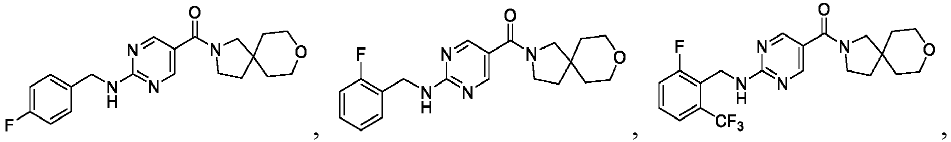

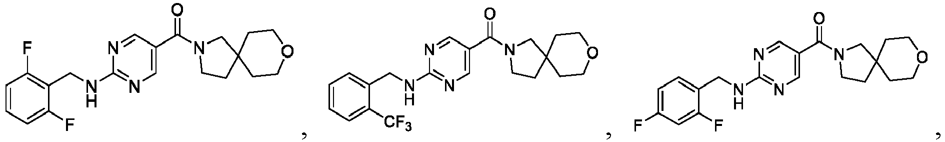

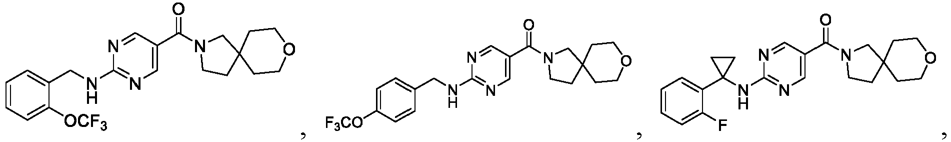

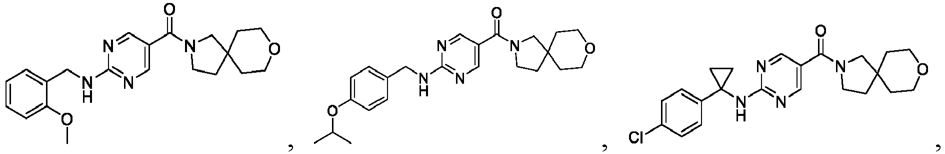

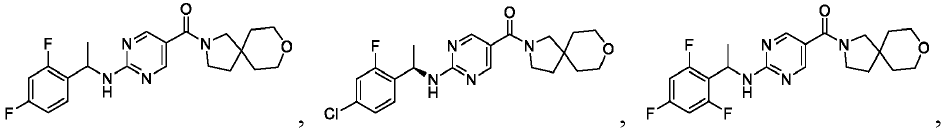

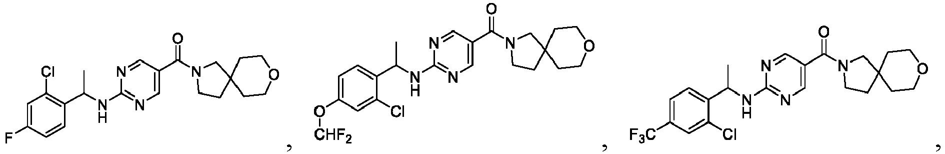

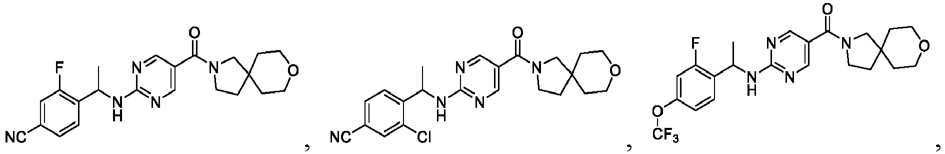

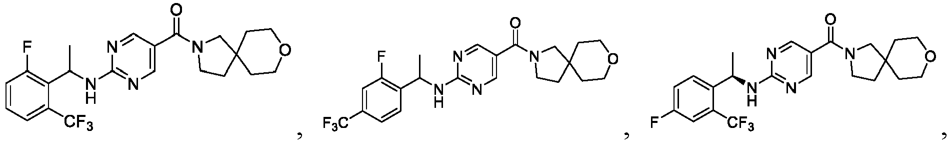

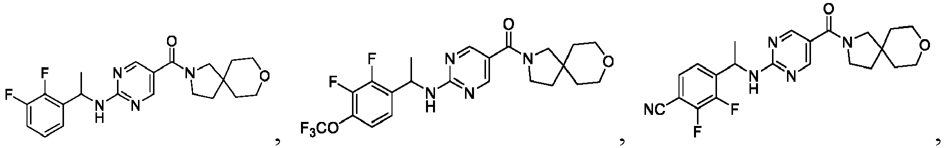

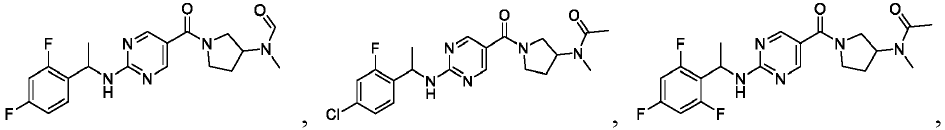

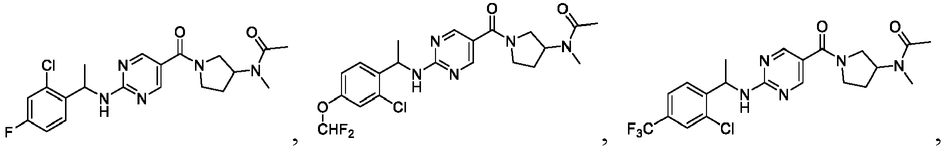

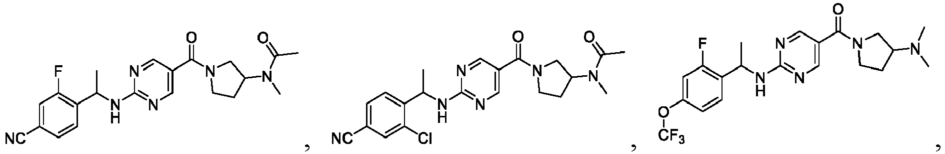

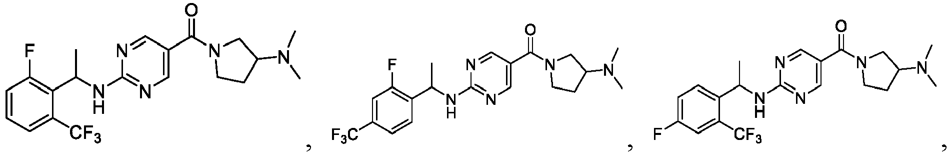

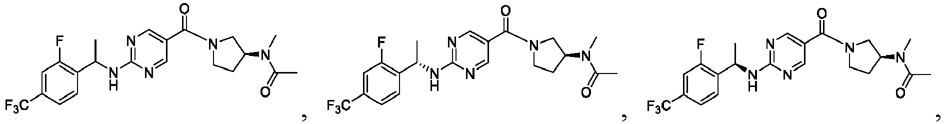

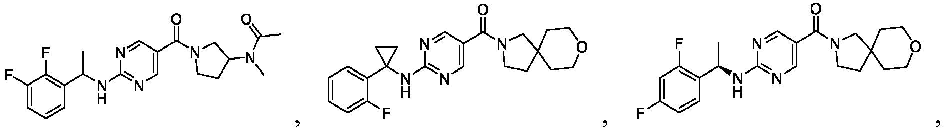

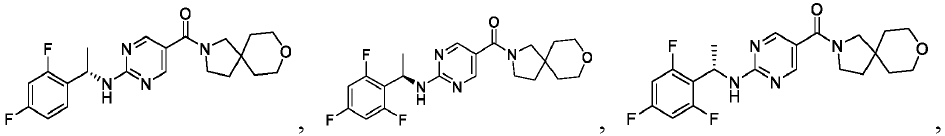

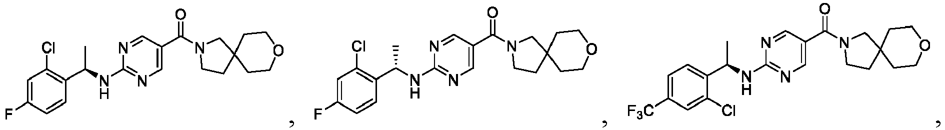

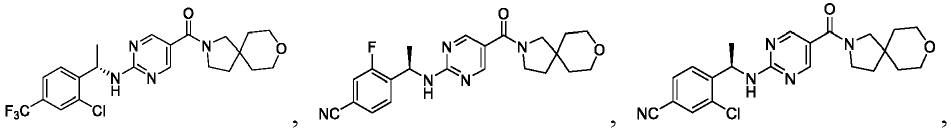

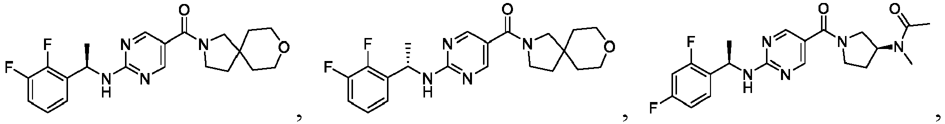

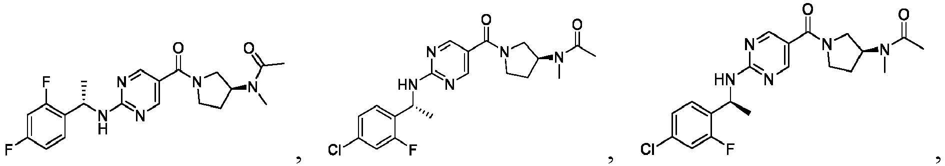

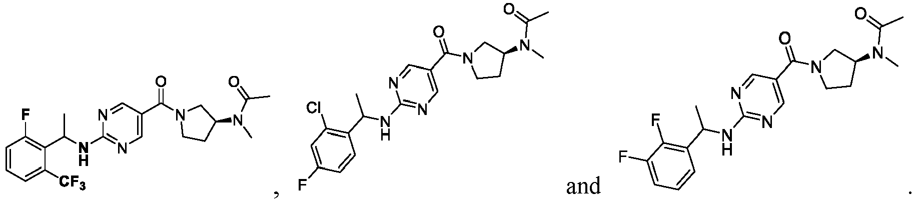

In the present disclosure, the pyrimidine carboxamide compound represented by formula I or the pharmaceutically acceptable salt thereof may have one or more chiral carbon atoms, and thus can be separated to obtain optically pure isomers, such as pure enantiomers, or racemates, or mixed isomers. Pure single isomers can be obtained by separation methods in the art, such as salt formation by chiral crystallization, or chiral preparative column separation.

In the present disclosure, if a stereoisomer of the pyrimidine carboxamide compound represented by formula I or the pharmaceutically acceptable salt thereof exists, the pyrimidine carboxamide compound may exist as a single stereoisomer or a mixture thereof (such as racemate). The term "stereoisomer" refers to *cis-trans* isomers or optical isomers. Such stereoisomers can be separated, purified and enriched by asymmetric synthetic methods or chiral separation methods (including but not limited to thin-layer chromatography, rotary chromatography, column chromatography, gas chromatography, high-pressure liquid chromatography, etc.), and can also be obtained by chiral resolution through bond formation (chemical bonding, etc.) or salt formation (physical bonding, etc.) with other chiral compounds. The term "single stereoisomer" means that the mass content of one stereoisomer of the compound of the present disclosure relative to all stereoisomers of the compound is not less than 95%.

Accordingly, throughout the description, those skill in the art may select the groups and substituents thereof in the pyrimidine carboxamide compounds represented by formula I, or the tautomers, mesomers, racemates, enantiomers, diastereomers thereof, or the mixture forms thereof, or the pharmaceutically acceptable salts thereof, so as to provide stable pyrimidine carboxamide compounds represented by formula I, or tautomers, mesomers, racemates, enantiomers, diastereomers thereof, or mixture forms thereof, or pharmaceutically acceptable salts thereof, including but not limited to the compounds in the embodiments of the present disclosure.

The pyrimidine carboxamide compounds represented by formula I, or the tautomers, mesomers, racemates, enantiomers, diastereomers thereof, or the mixture forms thereof, or the pharmaceutically acceptable salts thereof of the present disclosure may be synthesized by methods including the methods similar to those well known in the field of chemistry, whose steps and conditions can refer to the steps and conditions of similar reactions in the art, in particular according to the description herein. Starting materials are generally available from commercial sources such as Aldrich or can be readily prepared using methods well known to those skilled in the art (available via SciFinder and Reaxys online databases).

In the present disclosure, the pyrimidine carboxamide compounds represented by formula I, or the tautomers, mesomers, racemates, enantiomers, diastereomers thereof, or the mixture forms thereof, or the pharmaceutically acceptable salts thereof, may also be obtained through peripheral modification of the prepared pyrimidine carboxamide compounds represented by formula I, or the tautomers, mesomers, racemates, enantiomers, diastereomers thereof, or the mixture forms thereof, or the pharmaceutically acceptable salts thereof, using conventional methods in the art, so as to obtain the other pyrimidine carboxamide compounds represented by formula I, or the tautomers, mesomers, racemates, enantiomers, diastereomers thereof, or the mixture forms thereof, or the pharmaceutically acceptable salts thereof.

Generally, the compounds of the present disclosure can be prepared by the methods described herein, unless further specified, the definitions of substituents are as shown in formula I. The following reaction schemes and embodiments serve to further illustrate the present disclosure.

The raw materials or reagents necessary for the preparation of compounds represented by formula I are commercially available or can be prepared by synthetic methods known in the art. The compounds of the present disclosure can be prepared as a free base or as a salt thereof with the addition of an acid, as described in the experimental section below. The term pharmaceutically acceptable salt refers to a pharmaceutically acceptable salt as defined herein and has all the pharmaceutical activities of the parent compound. A pharmaceutically acceptable salt can be prepared by adding the corresponding acid to a suitable organic solvent of an organic base according to conventional methods.

Examples of salt formation include: salt formation with inorganic acids, such as hydrochloric acid, hydrobromic acid, sulfuric acid, nitric acid, phosphoric acid; and salt formation with organic acids, such as acetic acid, benzenesulfonic acid, benzoic acid, camphorsulfonic acid, citric acid, ethanesulfonic acid, fumaric acid, glucoheptonic acid, glutamic acid, glycolic acid, hydroxynaphthoic acid, 2-hydroxyethanesulfonic acid, lactic acid, maleic acid, malic acid, malonic acid, mandelic acid, methanesulfonic acid, hexadienedioic acid, 2-naphthalenesulfonic acid, propionic acid, salicylic acid, succinic acid, tartaric acid, *p-*toluenesulfonic acid or pivalic acid.

The pyrimidine carboxamide compound represented by formula I may have one or more chiral carbon atoms, and thus can be separated to obtain optically pure isomers, such as pure enantiomers, or racemates, or mixed isomers. Pure single isomers can be obtained by separation methods in the art, such as salt formation by chiral crystallization, or chiral preparative column separation.

The chemicals used in the synthetic route described herein include solvents, reagents, catalysts, protecting groups and deprotecting groups, and the protecting groups include *tert-*butoxycarbonyl (Boc). The above methods may additionally include steps before or after the steps specifically described herein, wherein suitable protecting groups may be added or removed to obtain the target compound. In addition, various synthetic steps can be performed alternately or sequentially to obtain the final target product.

The present disclosure provides a pharmaceutical composition, comprising the pyrimidine carboxamide compound represented by formula I, or the tautomer, mesomer, racemate, enantiomer, diastereomer thereof, or the mixture form thereof, or the pharmaceutically acceptable salt thereof, and, (one or more) pharmaceutical excipients (such as pharmaceutically available carriers, diluents, vehicles or other vehiculums). The dose of the pyrimidine carboxamide compound represented by formula I, or the tautomer, mesomer, racemate, enantiomer, diastereomer thereof, or the mixture form thereof, or the pharmaceutically acceptable salt thereof may be a therapeutically effective amount.

The present disclosure also provides a use of the pyrimidine carboxamide compound represented by formula I, or the tautomer, mesomer, racemate, enantiomer, diastereomer thereof, or the mixture form thereof, or the pharmaceutically acceptable salt thereof in the manufacture of a Vanin-1 inhibitor. In the use, the Vanin-1 inhibitor can be used *in vivo* in mammals; it can also be used *in vitro*, mainly for experimental purposes, for example, it can be used as a standard sample or control sample to provide a comparison, or made into a kit according to conventional methods in the art to provide a rapid detection for the inhibitory effect of Vanin-1. The term "Vanin-1 (enzyme) inhibitor" as used herein refers to a compound that binds to Vanin-1 (enzyme) and reduces the resulting enzyme activity.

The present disclosure also provides a use of the pyrimidine carboxamide compound represented by formula I, or the tautomer, mesomer, racemate, enantiomer, diastereomer thereof, or the mixture form thereof, or the pharmaceutically acceptable salt thereof in the manufacture of a drug; the drug may be used to prevent and/or treat diseases related to Vanin-1, or, the drug may be used to prevent and/or treat one or more of autoimmune diseases, inflammatory diseases, allergic diseases, metabolic diseases, infection-based diseases, fibrotic diseases, cardiovascular diseases, respiratory diseases, renal diseases, dermatological diseases, liver diseases, gastrointestinal diseases, oral diseases and hematopoietic diseases; for another example, a drug for Crohn's disease, inflammatory bowel disease and ulcerative colitis. The diseases related to Vanin-1 may comprise one or more of autoimmune diseases, inflammatory diseases, allergic diseases, metabolic diseases, infection-based diseases, fibrotic diseases, cardiovascular diseases, respiratory diseases, renal diseases, dermatological diseases, liver diseases, gastrointestinal diseases, oral diseases and hematopoietic diseases; for another example, inflammatory bowel disease, ulcerative colitis, Crohn's disease, colorectal cancer and gastritis.

Another aspect of the present disclosure relates to a method for preventing and/or treating diseases related to Vanin-1, comprising administering to a patient a therapeutically effective amount of the pyrimidine carboxamide compound represented by formula I, or the tautomer, mesomer, racemate, enantiomer, diastereomer thereof, or the mixture form thereof, or the pharmaceutically acceptable salt thereof, or the pharmaceutical composition comprising the same. For example, treatment of a disease or condition mediated by or otherwise associated with inhibition of Vanin-1 enzyme. For example, one or more of autoimmune diseases, inflammatory diseases, allergic diseases, metabolic diseases, infection-based diseases, fibrotic diseases, cardiovascular diseases, respiratory diseases, renal diseases, dermatological diseases, liver diseases, gastrointestinal diseases, oral diseases and hematopoietic diseases; for another example, inflammatory bowel disease, ulcerative colitis, Crohn's disease, colorectal cancer and gastritis.

Another aspect of the present disclosure relates to a method for preventing and/or treating one or more of autoimmune diseases, inflammatory diseases, allergic diseases, metabolic diseases, infection-based diseases, fibrotic diseases, cardiovascular diseases, respiratory diseases, renal diseases, dermatological diseases, liver diseases, gastrointestinal diseases, oral diseases and hematopoietic diseases (such as inflammatory bowel disease, ulcerative colitis, Crohn's disease, colorectal cancer and gastritis), comprising administering to a patient a therapeutically effective amount of the pyrimidine carboxamide compound represented by formula I, or the tautomer, mesomer, racemate, enantiomer, diastereomer thereof, or the mixture form thereof, or the pharmaceutically acceptable salt thereof, or the pharmaceutical composition comprising the same.

Another aspect of the present disclosure relates to a drug for inhibiting Vanin-1, comprising the pyrimidine carboxamide compound represented by formula I, or the tautomer, mesomer, racemate, enantiomer, diastereomer thereof, or the mixture form thereof, or the pharmaceutically acceptable salt thereof, or the pharmaceutical composition comprising the same.

The compounds of the present disclosure may be administered topically or systemically, for example, by enteral administration, such as rectal or oral administration, or by parenteral administration to mammals (in particular to humans). The compounds of the present disclosure may also be administered parenterally, for example, by inhalation, injection or infusion, such as by intravenous, intraarterial, intraosseous, intramuscular, intracerebral, extraventricular, intrasynovial, intrasternal, intrathecal, intralesional, intracranial, intratumoral, intradermal and subcutaneous injection or infusion.

The effective amount of the compounds, pharmaceutical compositions or drugs of the present disclosure depends on the species, body weight, age, individual condition, individual pharmacokinetic parameters, disease to be treated and mode of administration of mammals.

The effective amount of the compounds, pharmaceutical compositions or drugs of the present disclosure can be readily determined by conventional experiments, and the most effective and convenient route of administration and the most appropriate formulation can also be determined by conventional experiments.

The pharmaceutical excipients may be those widely used in the field of pharmaceutical production. Excipients are mainly used to provide a safe, stable and functional pharmaceutical composition, and may also provide a method to allow the active ingredients to dissolve at a desired rate after the subject receives administration, or to facilitate effective absorption of the active ingredients after the subject receives administration of the composition. The pharmaceutical excipients may be inert fillers, or provide a certain function, such as stabilizing the overall pH value of the composition or preventing the degradation of the active ingredients of the composition. The pharmaceutical excipients may comprise one or more of the following excipients: binders, suspending agents, emulsifiers, diluents, fillers, granulating agents, adhesives, disintegrants, lubricants, anti-adhesive agents, glidants, wetting agents, gelling agents, absorption retarders, dissolution inhibitors, enhancers, adsorbents, buffers, chelators, preservatives, coloring agents, corrigents and sweetening agents.

Substances which may be used as pharmaceutically acceptable excipients include, but are not limited to, ion exchangers, aluminum, aluminum stearate, lecithin, serum proteins such as human serum albumin, buffer substances such as phosphates, glycine, sorbic acid, potassium sorbate, partial glyceride mixture of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinylpyrrolidone, polyacrylates, waxes, polyethylene-polyoxypropylene block polymers, lanolin, sugars such as lactose, glucose and sucrose; starches such as corn starch and potato starch; cellulose and derivatives thereof such as sodium carboxymethyl cellulose, ethyl cellulose and cellulose acetate; gum powder; malt; gelatin; talc; excipients such as cocoa butter and suppository waxes; oils such as peanut oil, cottonseed oil, safflower oil, sesame oil, olive oil, corn oil and soybean oil; diol compounds such as propylene glycol and polyethylene glycol; esters such as ethyl oleate and ethyl laurate; agar; buffers such as magnesium hydroxide and aluminum hydroxide; alginic acid; pyrogen-free water; isotonic salts; Ringer's solution; ethanol, phosphate buffer solution, and other nontoxic suitable lubricants such as sodium dodecyl sulfate and magnesium stearate, coloring agents, release agents, coatings, sweetening agents, flavoring agents and fragrances, preservatives and antioxidants.

The pharmaceutical compositions of the present disclosure may be prepared according to the disclosure using any method known to those skilled in the art. For example, conventional mixing, dissolving, granulating, emulsifying, milling, encapsulating, entrapping or freeze-drying processes.

Pharmaceutical dosage forms of the compounds of the present disclosure may be provided in the form of immediate release, controlled release, sustained release or targeted drug release systems. Common dosage forms include, for example, solutions and suspensions, (micro)emulsions, ointments, gels and patches, liposomes, tablets, dragees, soft- or hard-shell capsules, suppositories, ovules, implants, amorphous or crystalline powders, aerosols and lyophilized formulations. Depending on the route of administration used, special devices may be needed to administer or give the drug, such as syringes and needles, inhalers, pumps, injection pens, applicators or specialflasks. Pharmaceutical dosage forms often consist of a drug, an excipient and a container/closure system. One or more excipients (also known as inactive ingredients) may be added to the compounds of the present disclosure to improve or facilitate the manufacture, stability, administration and safety of the drug, and may provide methods for obtaining the desired drug release profile. Therefore, the type of excipients added to the drug may depend on various factors such as the physical and chemical properties of the drug, route of administration, and preparation steps. Pharmaceutical excipients exist in the art and include those listed in various pharmacopoeias. (See U.S. Pharmacopoeia (USP), Japanese Pharmacopoeia (JP), European Pharmacopoeia (EP) and British Pharmacopoeia (BP); the U.S. Food and Drug Administration (www.fda.gov) Center for Drug Evaluation and Research (CEDR) publications such as "Inactive Ingredient Guide" (1996); "Hand book of Pharmaceutical Additives" by Ash and Ash (2002, Synapse Information Resources, Inc., Endicott NY; etc.).

Pharmaceutical dosage forms of the compounds of the present disclosure may be manufactured by any of the methods well known in the art, for example by conventional mixing, sieving, dissolving, melting, granulating, dragee-making, tabletting, suspending, extruding, spray-drying, milling, emulsifying, (nano-/micro-) encapsulating, entrapping or freeze-drying processes.

The pharmaceutical compositions of the present disclosure may be administered topically or systemically, for example, by enteral administration, such as rectal or oral administration, or by parenteral administration to mammals (in particular to humans), and comprise a therapeutically effective amount of the compound or the pharmaceutically acceptable salt thereof of the present disclosure as an active ingredient, together with a pharmaceutically acceptable excipient, such as a pharmaceutically acceptable carrier. The therapeutically effective amount of the active ingredients is as defined in the context, and depends on the species, body weight, age, individual condition, individual pharmacokinetic parameters, disease to be treated and mode of administration of mammals. For enteral administration, such as oral administration, the compounds of the present disclosure may be formulated into a wide variety of dosage forms.

The pharmaceutical compositions and dosage forms may comprise one or more compounds of the present disclosure or one or more pharmaceutically acceptable salts thereof as active ingredients. The pharmaceutically acceptable carrier may be a solid or a liquid. Formulations in solid form include powders, tablets, pills, lozenges, capsules, cachets, suppositories and dispersible granules. The solid carrier can also be one or more substances which act as diluents, flavoring agents, solubilizers, lubricants, suspending agents, binders, preservatives, tablet disintegrants or encapsulating materials. In powders, the carrier is usually a finely divided solid, which is a mixture with the finely divided active component. In tablets, the active component is usually mixed with a carrier having the necessary adhesive capacity in suitable proportions and compacted into the desired shape and size. Suitable carriers include, but are not limited to, magnesium carbonate, magnesium stearate, talc, sugar, lactose, pectin, dextrin, starch, gelatin, methyl cellulose, sodium carboxymethyl cellulose, low melting point wax, cocoa butter and the like. Formulations of the active compounds may comprise an encapsulating material as a carrier, providing a capsule in which the active component, with or without a carrier, is surrounded by a carrier bound thereto.

Other forms suitable for oral administration include formulations in liquid form, including emulsions, syrups, elixirs, aqueous solutions, aqueous suspensions, or formulations in solid form intended to be converted to formulations in liquid form shortly before use. Emulsions may be prepared in solutions, for example, in propylene glycol aqueous solutions, or may contain emulsifiers such as lecithin, sorbitan monooleate, or Arabic gum. Aqueous solutions may be prepared by dissolving the active component in water and adding suitable coloring agents, fragrances, stabilizers and thickening agents. Aqueous suspensions may be prepared by dispersing the finely divided active ingredients in water with binders such as natural or synthetic gums, resins, methyl cellulose, carboxymethyl cellulose and other commonly used suspending agents. Formulations in solid form include solutions, suspensions and emulsions, and may also contain coloring agents, fragrances, stabilizers, buffers, artificial and natural sweetening agents, dispersing agents, thickening agents, solubilizers and the like, in addition to the active component.

Exemplary compositions for rectal administration include suppositories, which may contain, for example, a suitable non-irritating excipient, such as cocoa butter, synthetic glyceride, or polyethylene glycol, which is a solid at room temperature, but melts and/or dissolves in the rectal lumen to release the drug.

The therapeutically effective amount may be first estimated using various methods well known in the art. The initial dose for animal studies can be based on the effective concentration established in cell culture assays. The dose range suitable for individuals can be determined, for example, using data obtained from animal studies and cell culture assays. In certain embodiments, the compounds of the present disclosure may be prepared as agents for oral administration.

The effective amount or therapeutically effective amount or dose of an agent (for example, the compound of the present disclosure) refers to the amount of the agent or compound that results in amelioration of symptoms or prolongation of survival in an individual. Toxicity and therapeutic efficacy of the molecules can be determined by standard pharmaceutical procedures in cell cultures or laboratory animals, for example by measuring the LD₅₀ (the dose lethal to 50% of the population) and the ED₅₀ (the dose therapeutically effective in 50% of the population). The dose ratio between toxic and therapeutic effects is the therapeutic index and it can be expressed as LD₅₀/ED₅₀. Agents which exhibit high therapeutic indices are preferred.

The effective amount or therapeutically effective amount refers to the amount of the compound or pharmaceutical composition that will elicit the biological or medical response in a tissue, system, animal, or human that is being sought by the researcher, veterinarian, physician, or other clinician. The dose is preferably within a range of circulating concentrations that include the ED₅₀ with little or no toxicity. The dose may vary within this range depending upon the dosage form utilized and/or the route of administration utilized. The proper formulation, route of administration, dose and interval of administration should be selected according to methods known in the art, taking into account the particularities of individual conditions.

The dose and interval may be individually adjusted to provide levels in plasma of the active moiety sufficient to obtain the desired effect; that is, the minimum effective concentration (MEC). The MEC will vary for each compound, but can be estimated, for example, from *in vitro* data and animal experiments. The dose necessary to obtain the MEC will depend on individual characteristics and route of administration. In cases of topical administration or selective uptake, the effective local concentration of the drug may not be related to plasma concentration.

The amount of the agent or composition administered may depend on various factors, including the sex, age and weight of the individual to be treated, the severity of the condition, the mode of administration, and the judgment of the prescribing physician.

If desired, the compositions of the present disclosure may be provided in packaging or dispensing devices containing one or more unit dosage forms (containing the active ingredients). For example, the packaging or dispensing device may comprise metal or plastic foil (such as foam packaging), or glass and rubber stoppers, such as in vials. The packaging or dispensing device may be accompanied by instructions for administration. Compositions comprising the compounds of the present disclosure formulated in compatible pharmaceutical carriers may also be prepared, placed in appropriate containers, and labeled for treatment of specified symptoms.

Unless otherwise specified, all technical and scientific terms as used herein have the standard meaning in the art to which the claimed subject matter pertains. If a term has more than one definition, the definition herein shall prevail.

### Group Definition

Unless otherwise specified, the following definitions as used herein shall apply. For the purposes of the present disclosure, the chemical elements correspond to the Periodic Table of the Elements, CAS Edition, and "Handbook of Chemistry and Physics", 75th Edition, 1994. In addition, general principles of organic chemistry can be found in the description of "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are incorporated herein by reference.

In the present description, groups and substituents thereof may be selected by those skilled in the art to provide stable structural moieties and compounds. When a substituent is described by a conventional chemical formula written from left to right, the substituent also includes the chemically equivalent substituent obtained when the structural formula is written from right to left.

Certain chemical groups defined herein are preceded by abbreviated notations to indicate the total number of carbon atoms present in the group. For example, C₁-C₆ alkyl refers to an alkyl having a total of 1, 2, 3, 4, 5 or 6 carbon atoms as defined below. The total number of carbon atoms in the abbreviated notation does not include the carbon that may be present in the substituent of the group.

The numerical range defined in the substituents of the present disclosure, such as 0-4, 1-4, 1-3 and the like, indicates integers within the range, for example, 1-6 refers to 1, 2, 3, 4, 5, 6.

In addition to the foregoing, when used in the description and claims of the present disclosure, the following terms have the meanings shown below unless otherwise specified.

The term "include" is open-ended, that is, it includes what is specified in the present disclosure, but does not exclude other aspects.

The term "substituted" means that one or more hydrogen atoms on a specific atom are substituted by the substituent, including deuterium and hydrogen variables, as long as the valence of the specific atom is normal and the substituted compound is stable.

In general, the term "substituted" means that one or more hydrogen atoms in a given structure are substituted by specific substituents. Further, when the group is substituted by the one or more substituents, the substituents are independent of each other, that is, the one or more substituents may be different from each other or identical. Unless otherwise specified, a substituent may substitute at each substitutable position of the group being substituted. When more than one position in a given structural formula can be substituted by one or more substituents selected from specific groups, then the substituents can be substituted equally or differently at each position.

In various sections of the description, the substituents of the disclosed compounds of the present disclosure are disclosed according to the type or range of the groups. In particular, it is noted that the present disclosure includes each independent sub-combination of the individual members of the types and ranges of such groups. For example, the term "C₁-C₆ alkyl" or "C₁₋₆ alkyl" specifically refers to independently disclosed methyl, ethyl, C₃ alkyl, C₄ alkyl, C₅ alkyl and C₆ alkyl; "C₁₋₄ alkyl" specifically refers to independently disclosed methyl, ethyl, C₃ alkyl (i.e. propyl, including n-propyl and isopropyl) and C₄ alkyl (i.e. butyl, including n-butyl, isobutyl, sec-butyl and tert-butyl).

The term "halogen" is selected from F, Cl, Br or I, and refers in particular to F or Cl.

In the present disclosure, the term "alkyl", as a group or part of another group, refers to a saturated aliphatic hydrocarbon group, which is a straight or branched chain group containing 1 to 12 carbon atoms, preferably a straight or branched chain alkyl containing 1 to 6 carbon atoms. The general formula is CₙH₂ₙ₊₁. The term "C₁-C₆ alkyl" means that the alkyl moiety contains 1, 2, 3, 4, 5 or 6 carbon atoms. In a certain embodiment, the term "alkyl" refers to C₁-C₆ alkyl. In a certain embodiment, the term "alkyl" refers to C₁-C₄ alkyl.

Non-limiting examples of lower alkyl containing 1 to 6 carbon atoms include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. Non-limiting examples of lower alkyl containing 1 to 12 carbon atoms include the above examples of lower alkyl containing 1 to 6 carbon atoms, as well as 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched chain isomers thereof and the like.

In the present disclosure, as a group or part of another group, unless otherwise specified, the term "cycloalkyl" refers to a saturated monocyclic, polycyclic or bridged carbocyclic substituent consisting only of carbon atoms and hydrogen atoms, and it may be connected to the rest of the molecule by a single bond via any suitable carbon atom; in the case of a polycyclic cycloalkyl, it may be a bridged ring or spiro ring system with a fused ring or spiro ring connection (that is, two gem-hydrogens on a carbon atom are substituted by an alkylene). Cycloalkyl substituents may be connected to the central molecule via any suitable carbon atom. In some embodiments, a ring having 3 to 10 carbon atoms may be represented as C₃-C₁₀ cycloalkyl. In some embodiments, C₃-C₆ cycloalkyl includes cyclopropyl (C₃), cyclobutyl (C₄), cyclopentyl (C₅) and cyclohexyl (C₆). In some embodiments, examples of C₃-C₁₀ cycloalkyl include the above C₃-C₆ cycloalkyl groups together with cycloheptyl (C₇), cyclooctyl (C₈), cyclononyl (C₉) and cyclodecyl (C₁₀).

In the present disclosure, the term "heterocycloalkyl", as a group or part of another group, refers to a stable 3- to 7-membered saturated cyclic group consisting of 2 to 6 carbon atoms as well as 1 to 4 heteroatoms selected from nitrogen, oxygen and sulfur. Exemplary 3-membered heterocycloalkyl includes, but is not limited to, aziridinyl, oxiranyl, and thiacyclopropanyl, or a stereoisomer thereof; exemplary 4-membered heterocycloalkyl includes, but is not limited to, azetidinyl, epoxypropanyl, thietanyl, or an isomer and a stereoisomer thereof; exemplary 5-membered heterocycloalkyl includes, but is not limited to, tetrahydrofuranyl, tetrahydrothienyl, pyrrolidinyl, thiazolidinyl, isothiazolidinyl, oxazolidinyl, isoxazolidinyl, imidazolidinyl, pyrazolidinyl, dioxolanyl, oxathiofuranyl, dithiofuranyl, or an isomer and a stereoisomer thereof. Exemplary 6-membered heterocycloalkyl includes, but is not limited to, piperidinyl, tetrahydropyranyl, thiacyclopentyl, morpholinyl, thiomorpholinyl, dithianyl, dioxanyl, piperazinyl, triazinyl, or an isomer and a stereoisomer thereof; exemplary 7-membered heterocycloalkyl includes, but is not limited to, azepanyl, oxepanyl, thiepanyl, and diazepanyl, or an isomer and a stereoisomer thereof. In a certain embodiment, "heterocycloalkyl" is C₂-C₅ heterocycloalkyl, wherein the heteroatom is selected from one or more of N, O and S, and the number of heteroatoms is 1, 2 or 3.

The terms "moiety", "structural moiety", "chemical moiety", "group", "chemical group" as used herein refer to a specific fragment or functional group in a molecule. Chemical moieties are generally considered to be chemical entities embedded or attached to molecules.

When the enumerative substituent does not indicate by which atom it is linked to the compound included but not specifically mentioned in a general chemical formula, such substituent can be bonded by any atom thereof. A combination of the substituent and/or the variant thereof is allowed only when the combination results in a stable compound.

In various sections of the present disclosure, linking substituents are described. When the structure clearly requires a linking group, the enumerative Markush variables for the group should be understood as linking groups. For example, if the structure requires a linking group and the Markush group definition for the variable enumerates "alkyl", it should be understood that the "alkyl" represents a linked alkylene group.

In some specific structures, when an alkyl group is clearly indicated as a linking group, the alkyl group represents a linked alkylene group, for example, C₁-C₆ alkyl in the group "halo-C₁-C₆ alkyl" should be understood as C₁-C₆ alkylene.

The term "alkylene" means a saturated divalent hydrocarbyl group obtained by removing two hydrogen atoms from a saturated straight or branched chain hydrocarbyl. Examples of alkylene groups include methylene (-CH₂-), ethylene {including -CH₂CH₂- or - CH(CH₃)-}, isopropylidene {including -CH(CH₃)CH₂- or -C(CH₃)₂-} and the like.

Unless otherwise specified, all technical and scientific terms as used herein have the standard meaning in the art to which the claimed subject matter pertains. If a term has more than one definition, the definition herein shall prevail.

Unless otherwise specified, it should be understood that the singular form used in the present disclosure, such as "a", includes plural referents. In addition, the term "include" is open-ended and not closed, that is, it includes what is specified in the present disclosure, but does not exclude other aspects.

Unless otherwise specified, the present disclosure uses the conventional methods of mass spectrometry and elemental analysis, and the steps and conditions can be referred to the conventional operating steps and conditions in the art.

Unless otherwise specified, the present disclosure uses standard nomenclature and standard laboratory procedures and techniques of analytical chemistry, organic synthetic chemistry and optics. In some cases, standard techniques are used for chemical synthesis, chemical analysis, and performance testing of luminescent devices.

In addition, it should be noted that, unless otherwise clearly specified, the description "...be independently" used in the present disclosure should be understood in a broad sense, which means that the described individuals are independent of each other and can be the same or different specific groups independently. In more detail, the description "...be independently" can mean either that the specific options expressed between the same symbols in different groups do not affect each other; or that the specific options expressed between the same symbols in the same group do not affect each other.

It can be understood by those skilled in the art that, according to the convention used in the art, " 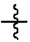 " used in the structural formula of the group described in the present disclosure means that the corresponding group is linked to other fragments and groups in the compound through this site.

"Pharmaceutically acceptable" means that which is useful in preparing a pharmaceutical composition that is generally safe and non-toxic, and is not biologically and otherwise undesirable, and includes that which is pharmaceutically acceptable for veterinary use as well as human use.

The term "excipient" refers to a pharmaceutically acceptable chemical substance, such as an agent known to those of ordinary skill in the pharmaceutical field for use in facilitating the administration of a drug. It is a compound that is useful in preparing a pharmaceutical composition that is generally safe and non-toxic, and is biologically or otherwise undesirable, and includes excipients that are pharmaceutically acceptable for veterinary use as well as human use. Typical excipients include binders, surfactants, diluents, disintegrants and lubricants.

The term "therapeutically effective amount" refers to the amount of a compound used which, when administered to a subject to treat a disease state, is sufficient to achieve such treatment of the disease state. The "therapeutically effective amount" will vary according to the compound, the disease state being treated, the severity of the disease being treated, the age and relative health of the subject, the route and mode of administration, the judgment of the attending physician or veterinarian, and the like.

The term "mammal" refers to a human or any mammal such as a primate, farm animal, pet animal or laboratory animal. Examples of such animals are monkeys, cows, sheep, horses, pigs, dogs, cats, rabbits, mice and rats, and the like. Mammals are preferably humans.

The above preferred conditions can be combined arbitrarily to obtain preferred embodiments of the present disclosure without violating common knowledge in the art.

The reagents and raw materials used in the present disclosure are all commercially available.

The positive effect of the present disclosure is that a pyrimidine carboxamide compound is provided, which can be used as an inhibitor of Vanin enzyme, especially an inhibitor of Vanin-1; and it can be used to prevent and/or treat Crohn's disease, ulcerative colitis and inflammatory bowel disease.

### DETAILED DESCRIPTION

The present disclosure is further illustrated below by means of embodiments, but the present disclosure is not thereby limited to the scope of the embodiments. Experimental methods for which specific conditions are not specified in the following embodiments are selected according to conventional methods and conditions, or according to the trade description.

### Example 1

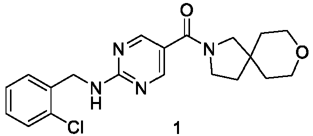

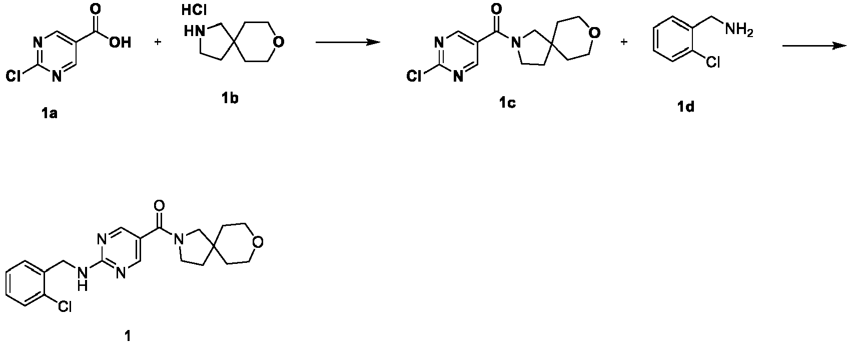

### Step 1

To a solution of compound **1a** (25.40 g), compound **1b** (31.00 g) and triethylamine (61.00 g) in acetonitrile (250 mL) was slowly added dropwise T₃P (propylphosphonic anhydride) (254.00 g) at -10°C under nitrogen atmosphere. After the dropwise addition was completed, the reaction system was kept at -5°C and reacted for 3 hours. The reaction was completed. The reaction mixture was quenched with water (300 mL), concentrated to remove the organic solvent, and the residue was stirred at 5°C for 1 hour. A solid was precipitated, then the mixture was filtered, and the filter cake was washed with water (100 mL × 1) and dried to obtain compound **1c** (42.00 g) with a yield of 93%.

¹H NMR (400 MHz, CDCl₃) δ 8.80 (s, 2H), 3.81-3.57 (m, 7H), 3.36 (s, 1H), 1.93 (td, *J* = 14.6, 7.2 Hz, 2H), 1.66 (t, *J* = 5.4 Hz, 2H), 1.58 (dd, *J* = 11.1, 4.6 Hz, 2H).

### Step 2

A solution of compound **1d** (400 mg, 2.82 mmol), **1c** (792.5 mg, 2.82 mmol) and *N*,*N-*diisopropylethylamine (2 mL) in 1,4-dioxane (30 mL) was heated to 100°C and reacted overnight. After the reaction was completed, the reaction mixture was cooled to room temperature, concentrated under reduced pressure, and the obtained residue was purified by silica gel column (methanol/dichloromethane = 0-100%) to obtain compound **1** (356 mg, yield: 33%) as an off-white solid.

¹H NMR (400 MHz, DMSO-*d₆*) δ 8.55 (s, 2H), 8.23 (brs, 1H), 7.29-7.47 (m, 4H), 4.67-4.61 (m, 2H), 3.53-3.64 (m, 8H), 1.80 (m, 2H), 1.45-1.55 (m, 4H).

LCMS (ESI), [M+H]⁺ = 387.1

### Example 2

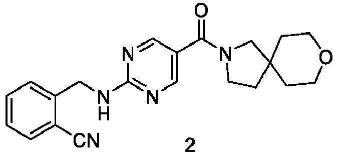

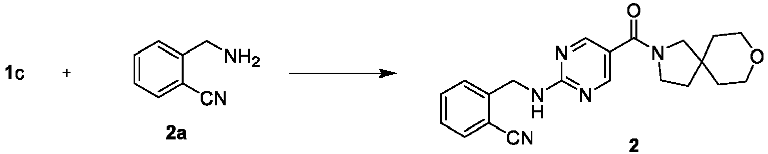

### Step 1

Compound **2** (10 mg, yield: 4%) was obtained from compounds **1c** and **2a** according to the method of Example **1.**

¹H NMR (400 MHz, DMSO-*d₆*) δ 10.7 (brs, 1H), 9.04-9.06 (m, 2H), 8.30 (m, 1H), 7.79 (m 2H), 7.65 (m, 1H), 5.33 (s, 2H), 3.44-3.65 (m, 8H), 1.83-1.88 (m, 2H), 1.46-1.49 (m, 4H).

LCMS (ESI), [M+H]⁺ = 378.1

### Example 3

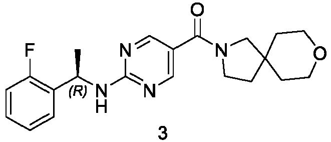

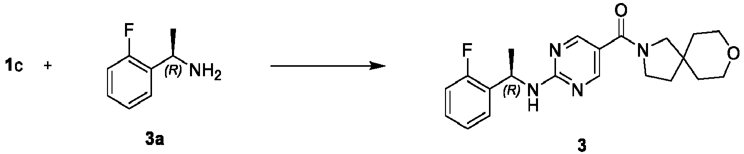

### Step 1

Compound **3** (150 mg, yield: 36%) was obtained from compounds **1c** and **3a** according to the method of Example **1.**

¹H NMR (400 MHz, DMSO-*d₆*) δ 8.50 (s, 2H), 8.27-8.29(m, 1H), 7.49-7.43 (m, 1H), 7.25-7.27(m, 1H), 7.11-7.16 (m, 2H), 5.42 (m, 1H), 3.51-3.59(m, 6H), 3.42 (m, 2H), 1.76-1.78(m, 2H), 1.44-1.52 (m, 7H).

LCMS (ESI), [M+H]⁺ = 385.2

### Example 4

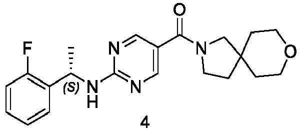

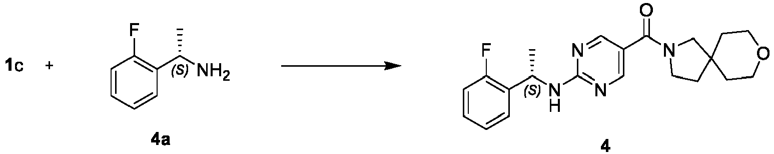

### Step 1

Compound **4** (105 mg, yield: 25%) was obtained from compounds **1c** and **4a** according to the method of Example **1.**

¹H NMR (400 MHz, DMSO-*d₆*) δ 8.49 (s, 2H), 8.27-8.29(m, 1H), 7.49-7.43 (m, 1H), 7.25-7.27(m, 1H), 7.11-7.16 (m, 2H), 5.42 (m, 1H), 3.40-3.59(m, 8H), 1.76-1.78(m, 2H), 1.23-1.52 (m, 7H).

LCMS (ESI), [M+H]⁺ = 385.2

### Example 5

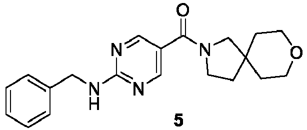

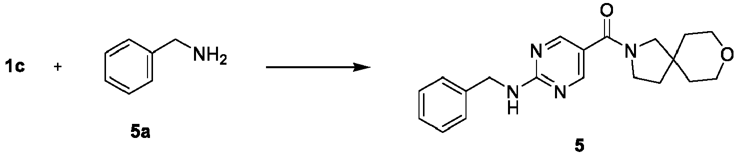

### Step 1

Compound **5** (57 mg, yield: 45%) was obtained from compounds **1c** and **5a** according to the method of Example **1.**

¹H NMR (400 MHz, DMSO-*d₆*) δ 8.52 (s, 2H), 8.21 (s, 1H), 7.31 (d, J = 4.4 Hz, 4H), 7.26-7.17 (m, 1H), 4.55 (m, 2H), 3.68-3.35 (m, 8H), 1.78 (m, 2H), 1.53-1.45 (m, 4H).

LCMS (ESI), [M+H]⁺ = 353.2

### Example 6

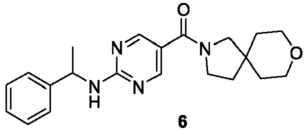

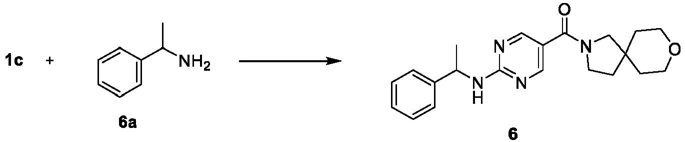

### Step 1

Compound **6** (27 mg, yield: 30%) was obtained from compounds **1c** and **6a** according to the method of Example **1.**

¹H NMR (400 MHz, DMSO-*d₆*) δ 8.48 (s, 2H), 8.22 (m, 1H), 7.38 (m, 2H), 7.30 (m, 2H), 7.19 (m, 1H), 5.16 (m, 1H), 3.66-3.34 (m, 8H), 1.77 (m, 2H), 1.52 (s, 1H), 1.45 (m, 5H), 1.24 (s, 1H).

LCMS (ESI), [M+H]⁺ = 367.2

### Example 7

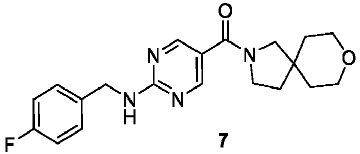

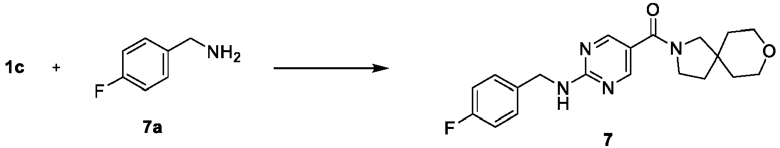

### Step 1

Compound **7** (37 mg, yield: 28%) was obtained from compounds **1c** and **7a** according to the method of Example **1.**

¹H NMR (400 MHz, DMSO-*d₆*) δ 8.52 (m, 2H), 8.22 (s, 1H), 7.45-7.27 (m, 2H), 7.21-6.95 (m, 2H), 4.52 (m, 2H), 3.70-3.34 (m, 8H), 1.78 (m, 2H), 1.49 (m, 4H).

LCMS (ESI), [M+H]⁺ = 371.1

### Example 8

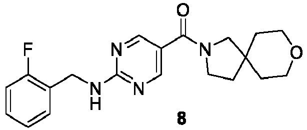

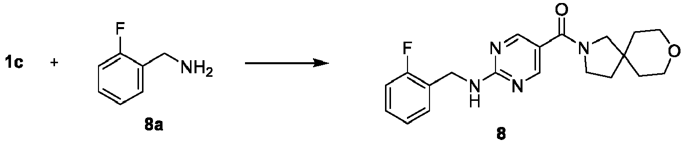

### Step 1

Compound **8** (29 mg, yield: 30%) was obtained from compounds **1c** and **8a** according to the method of Example **1.**

¹H NMR (400 MHz, DMSO-*d₆*) δ 8.53 (m, 2H), 8.19 (s, 1H), 7.44-7.23 (m, 2H), 7.23-7.07 (m, 2H), 4.59 (m, 2H), 3.69-3.35 (m, 8H), 1.78 (m, 2H), 1.54-1.44 (m, 4H).

LCMS (ESI), [M+H]⁺ = 371.1

### Example 9

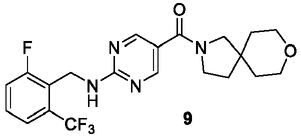

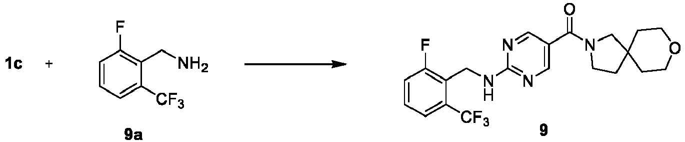

### Step 1

Compound **9** (43 mg, yield: 47%) was obtained from compounds **1c** and **9a** according to the method of Example **1.**

¹H NMR (400 MHz, DMSO-*d₆*) δ 8.56 (s, 2H), 7.95 (s, 1H), 7.60 (m, 3H), 4.65 (m, 2H), 3.72-3.41 (m, 7H), 1.80 (s, 2H), 1.50 (m, 4H), 1.24 (s, 1H).

LCMS (ESI), [M+H]⁺ = 439.1

### Example 10

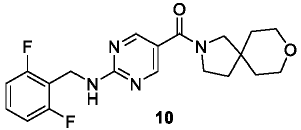

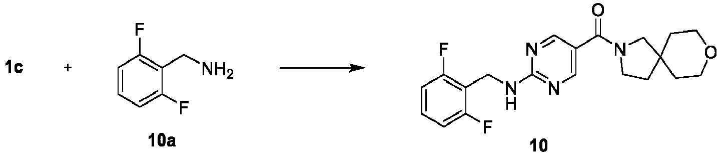

### Step 1

Compound **10** (53 mg, yield: 64%) was obtained from compounds **1c** and **10a** according to the method of Example **1.**

¹H NMR (400 MHz, DMSO-*d₆*) δ 8.53 (s, 2H), 8.03 (s, 1H), 7.38 (m, 1H), 7.08 (m, 2H), 4.59 (m, 2H), 3.81-3.38 (m, 7H), 1.79 (s, 2H), 1.49 (m, 4H).

LCMS (ESI), [M+H]⁺ = 389.2

### Example 11

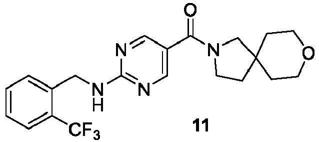

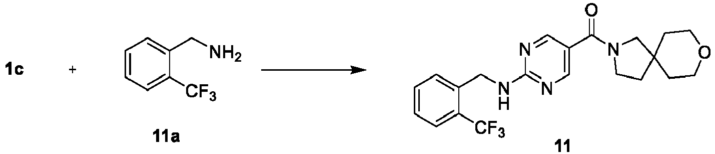

### Step 1

Compound **11** (51 mg, yield: 56%) was obtained from compounds **1c** and **11a** according to the method of Example **1.**

¹H NMR (400 MHz, DMSO-*d₆*) δ 8.52 (m, 2H), 8.22 (s, 1H), 7.45-7.27 (m, 2H), 7.21-6.95 (m, 2H), 4.52 (m, 2H), 3.70-3.34 (m, 7H), 1.78 (m, 2H), 1.49 (m, 4H).

LCMS (ESI), [M+H]⁺ = 421.2.

### Example 12

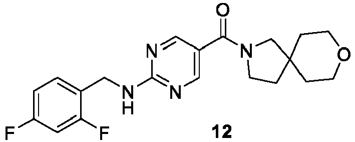

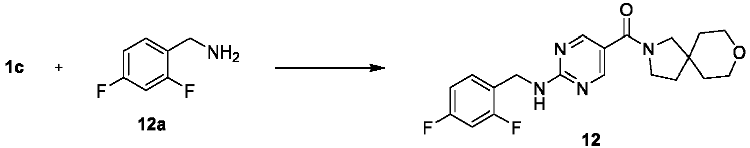

### Step 1

Compound **12** (55 mg, yield: 66%) was obtained from compounds **1c** and **12a** according to the method of Example **1.**

¹H NMR (400 MHz, DMSO-*d₆*) δ 8.53 (s, 2H), 8.19 (s, 1H), 7.39 (m, 1H), 7.21 (m, 1H), 7.04 (m, 1H), 4.55 (m, 2H), 3.67 - 3.35 (m, 8H), 1.78 (m, 2H), 1.49 (m, 4H).

LCMS (ESI), [M+H]⁺ = 389.1

### Example 13

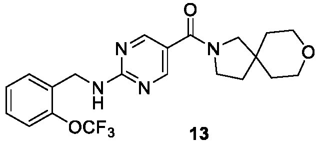

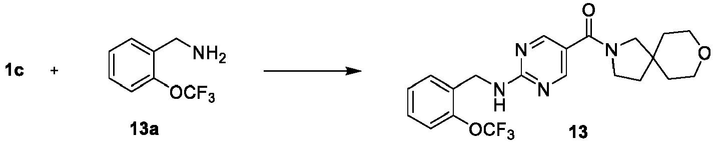

### Step 1

Compound **13** (58 mg, yield: 62%) was obtained from compounds **1c** and **13a** according to the method of Example **1.**

¹H NMR (400 MHz, DMSO-*d₆*) δ 8.54 (s, 2H), 8.20 (s, 1H), 7.53-7.28 (m, 4H), 4.64 (m, 2H), 3.73 -3.39 (m, 8H), 1.80 (s, 2H), 1.50 (m, 4H).

LCMS (ESI), [M+H]⁺ = 437.1

### Example 14

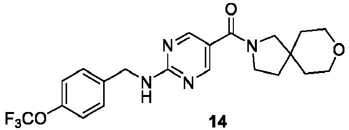

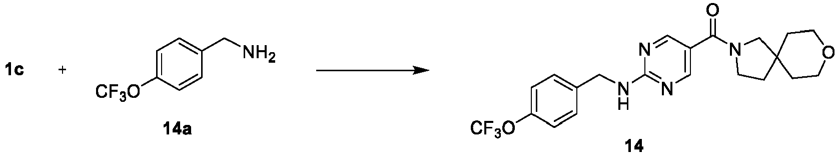

### Step 1

Compound **14** (29 mg, yield: 31%) was obtained from compounds **1c** and **14a** according to the method of Example **1.**

¹H NMR (400 MHz, DMSO-*d₆*) δ 8.52 (s, 2H), 8.25 (s, 1H), 7.43 (m, 2H), 7.30 (m, 2H), 4.57 (m, 2H), 3.59-3.45 (m, 8H), 1.78 (s, 2H), 1.55-1.43 (m, 4H).

LCMS (ESI), [M+H]⁺ = 437.1

### Example 15

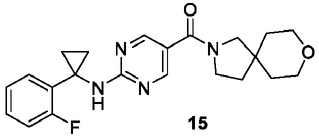

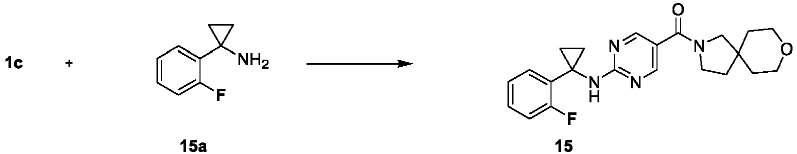

### Step 1

Compound **15** (83 mg, yield: 98%) was obtained from compounds **1c** and **15a** according to the method of Example **1.**

¹H NMR (400 MHz, DMSO-*d₆*) δ 8.49 (m, 3H), 7.62 (m, 1H), 7.30-7.19 (m, 1H), 7.10 (m, 2H), 3.52 (m, 8H), 1.78 (s, 2H), 1.49 (m, 4H), 1.26 (m, 2H), 1.18 (m, 2H).

LCMS (ESI), [M+H]⁺ = 397.2

### Example 16

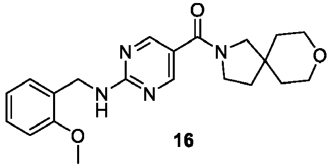

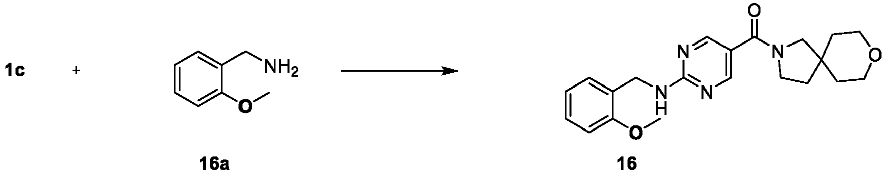

### Step 1

Compound **16** (57 mg, yield: 71%) was obtained from compounds **1c** and **16a** according to the method of Example **1.**

¹H NMR (400 MHz, DMSO-*d₆*) δ 8.53 (s, 2H), 8.00 (s, 1H), 7.29-7.20 (m, 1H), 7.18 (s, 1H), 6.99 (m, 1H), 6.89 (m, 1H), 4.53 (m, 2H), 3.84 (m, 3H), 3.70-3.39 (m, 8H), 1.80 (s, 2H), 1.51 (m, 4H).

LCMS (ESI), [M+H]⁺ = 383.2

### Example 17

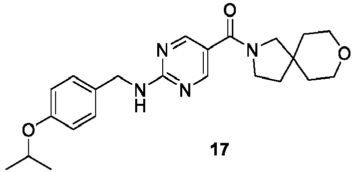

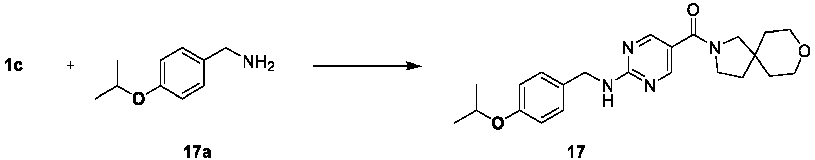

### Step 1

Compound **17** (61 mg, yield: 70%) was obtained from compounds **1c** and **17a** according to the method of Example **1.**

¹H NMR (400 MHz, DMSO-*d₆*) δ 8.51 (s, 2H), 8.12 (s, 1H), 7.21 (m, 2H), 6.87-6.79 (m, 2H), 4.55 (m, 1H), 4.46 (m, 2H), 3.66-3.43 (m, 8H), 1.79 (s, 2H), 1.49 (m, 4H), 1.23 (m, 6H).

LCMS (ESI), [M+H]⁺ = 411.2

### Example 18

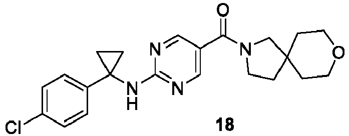

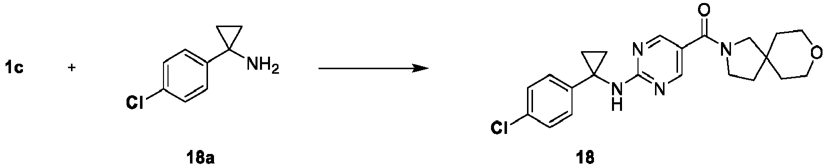

Compound **18** (15 mg, yield: 17%) was obtained from compounds **1c** and **18a** according to the method of Example **1.**

¹H NMR (400 MHz, DMSO-*d₆*) δ 8.55 (m, 3H), 7.34-7.09 (m, 4H), 3.68-3.48 (m, 8H), 1.78 (m, 2H), 1.49 (m, 4H), 1.30-1.24 (m, 3H), 1.19 (m, 1H).

LCMS (ESI), [M+H]⁺ = 413.1

### Example 19

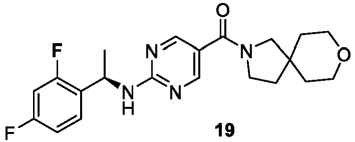

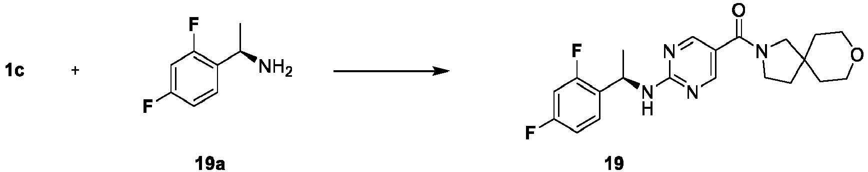

Compound **19** (46 mg) was obtained from compounds **1c** and **19a** according to the method of Example **1.**

¹H NMR (400 MHz, DMSO-*d₆*) δ 8.51 (s, 2H), 8.33-8.26 (m, 1H), 7.53-7.46 (m, 1H), 7.20-7.16 (m, 1H), 7.10-7.02 (m, 1H), 5.38 (brs, 1H), 3.66-3.40 (m, 8H), 1.83-1.70 (m, 2H), 1.58-1.35 (m, 7H).

LCMS (ESI), [M+H]⁺ = 403.2

### Example 20

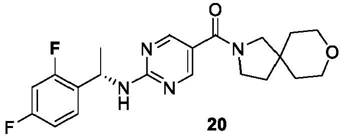

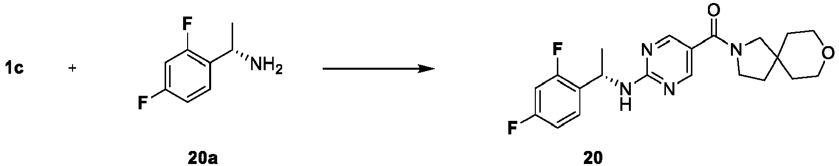

Compound **20** (55 mg) was obtained from compounds **1c** and **20a** according to the method of Example **1.**

¹H NMR (400 MHz, DMSO-*d₆*) δ 8.51 (s, 2H), 8.33-8.26 (m, 1H), 7.53-7.46 (m, 1H), 7.20-7.16 (m, 1H), 7.10-7.02 (m, 1H), 5.38 (brs, 1H), 3.66-3.40 (m, 8H), 1.83-1.70 (m, 2H), 1.58-1.35 (m, 7H).

LCMS (ESI), [M+H]⁺ = 403.2

### Example 21

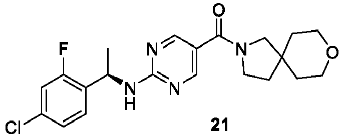

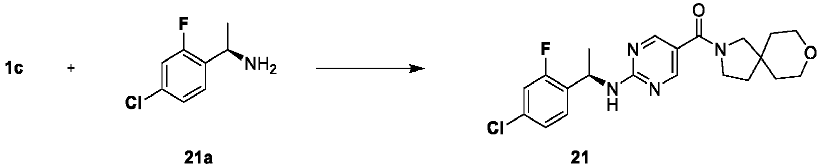

Compound **21** (50 mg) was obtained from compounds **1c** and **21a** according to the method of Example **1.**

¹H NMR (400 MHz, DMSO-*d₆*) δ 8.51 (s, 2H), 8.34-8.30 (m, 1H), 7.49-7.43 (m, 1H), 7.40-7.37 (m, 1H), 7.29-7.25 (m, 1H), 5.36 (brs, 1H), 3.66-3.40 (m, 8H), 1.83-1.70 (m, 2H), 1.58-1.35 (m, 7H).

LCMS (ESI), [M+H]⁺ = 419.1

### Example 22

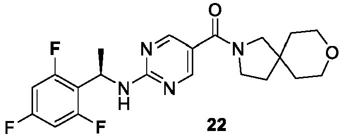

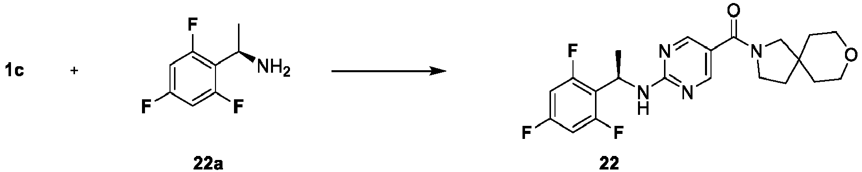

Compound **22** (16 mg) was obtained from compounds **1c** and **22a** according to the method of Example **1.**

¹H NMR (400 MHz, DMSO-*d₆*) δ 8.48 (s, 2H), 8.17-8.16 (m, 1H), 7.12-7.08 (m, 2H), 5.36-5.33 (m, 1H), 3.60-3.37 (m, 8H), 1.78-1.76 (m, 2H), 1.55-1.37 (m, 7H).

LCMS (ESI), [M+H]⁺ = 421.2

### Example 23

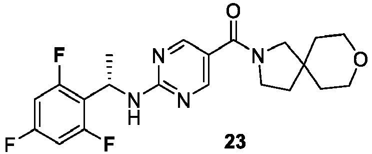

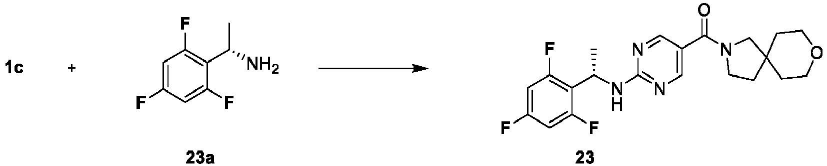

Compound **23** (48 mg) was obtained from compounds **1c** and **23a** according to the method of Example **1.**

¹H NMR (400 MHz, DMSO-*d₆*) δ 8.48 (s, 2H), 8.17-8.16 (m, 1H), 7.12-7.08 (m, 2H), 5.36-5.33 (m, 1H), 3.60-3.36 (m, 8H), 1.78-1.75 (m, 2H), 1.56-1.37 (m, 7H).

LCMS (ESI), [M+H]⁺ = 421.2

### Example 24

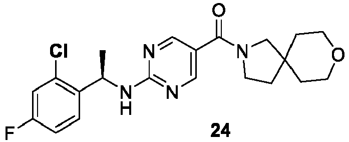

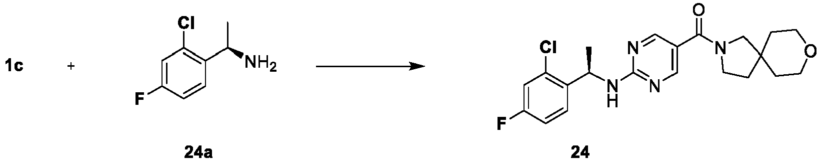

Compound **24** (32 mg) was obtained from compounds **1c** and **24a** according to the method of Example **1.**

¹H NMR (400 MHz, DMSO-*d₆*) δ 8.50 (s, 2H), 8.40-8.35 (m, 1H), 7.57-7.53 (m, 1H), 7.40-7.37 (m, 1H), 7.23-7.18 (m, 1H), 5.43-5.39 (m, 1H), 3.61-3.40 (m, 8H), 1.81-1.74 (m, 2H), 1.56-1.37 (m, 7H).

LCMS (ESI), [M+H]⁺ = 419.1

### Example 25

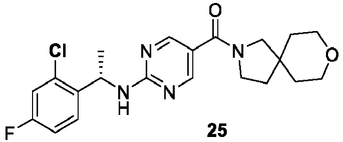

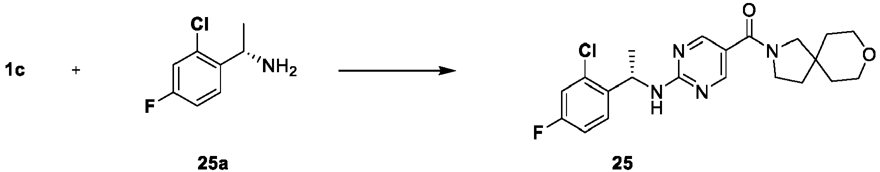

Compound **25** (31 mg) was obtained from compounds **1c** and **25a** according to the method of Example **1.**

¹H NMR (400 MHz, DMSO-*d₆*) δ 8.49 (s, 2H), 8.38-8.34 (m, 1H), 7.55 (brs, 1H), 7.39-7.37 (m, 1H), 7.22-7.17 (m, 1H), 5.41 (brs, 1H), 3.60-3.36 (m, 8H), 1.80-1.74 (m, 2H), 1.56-1.37 (m, 7H).

LCMS (ESI), [M+H]⁺ = 419.1

### Example 26

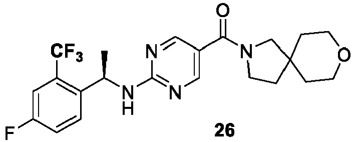

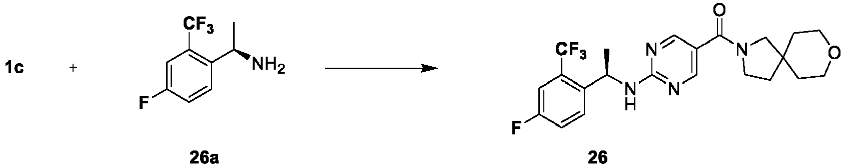

Compound **26** (43 mg) was obtained from compounds **1c** and **26a** according to the method of Example **1.**

¹H NMR (400 MHz, DMSO-*d₆*) δ 8.50-8.35 (m, 3H), 7.91-7.87 (m, 1H), 7.58-7.51 (m, 2H), 5.50-5.39 (m, 1H), 3.61-3.40 (m, 8H), 1.81-1.72 (m, 2H), 1.56-1.37 (m, 7H).

LCMS (ESI), [M+H]⁺ = 453.2.

### Example 27

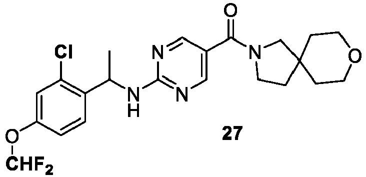

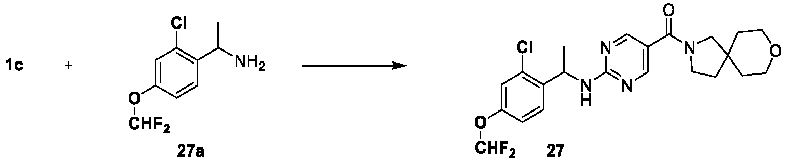

Compound **27** (67 mg) was obtained from compounds **1c** and **27a** according to the method of Example **1.**

¹H NMR (400 MHz, DMSO-*d₆*) δ 8.50-8.35 (m, 3H), 7.57-7.55 (m, 1H), 7.30-7.15 (m, 3H), 5.50-5.39 (m, 1H), 3.63-3.40 (m, 8H), 1.81-1.72 (m, 2H), 1.56-1.37 (m, 7H).

LCMS (ESI), [M+H]⁺ = 467.1.

### Example 28

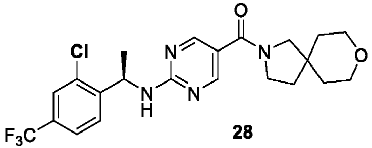

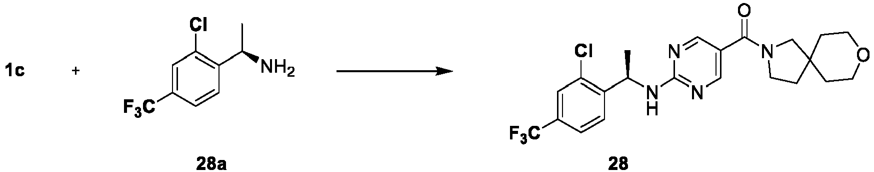

Compound **28** (41 mg) was obtained from compounds **1c** and **28a** according to the method of Example **1.**

¹H NMR (400 MHz, DMSO-*d₆*) δ 8.55-8.35 (m, 3H), 7.83 (s, 1H), 7.72 (brs, 2H), 5.50-5.39 (m, 1H), 3.61-3.40 (m, 8H), 1.81-1.71 (m, 2H), 1.56-1.37 (m, 7H).

LCMS (ESI), [M+H]⁺ = 469.1.

### Example 29

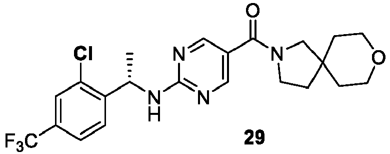

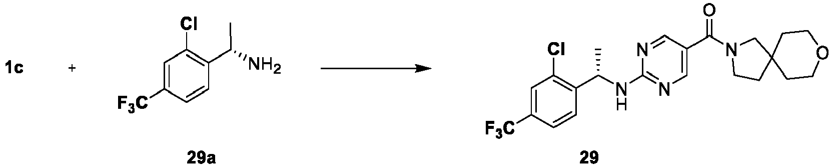

Compound **29** (33 mg) was obtained from compounds **1c** and **29a** according to the method of Example **1.**

¹H NMR (400 MHz, DMSO-*d₆*) δ 8.55-8.35 (m, 3H), 7.82 (s, 1H), 7.72 (brs, 2H), 5.50-5.39 (m, 1H), 3.61-3.40 (m, 8H), 1.81-1.71 (m, 2H), 1.56-1.37 (m, 7H).

LCMS (ESI), [M+H]⁺ = 469.1.

### Example 30

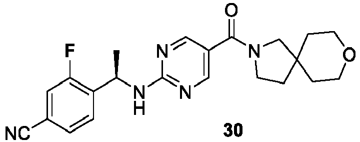

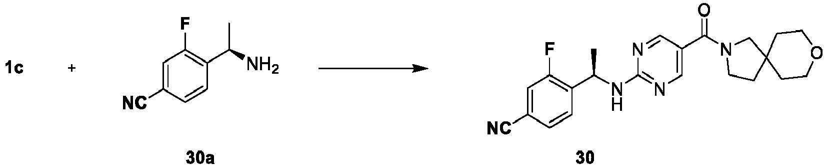

Compound **30** (16 mg) was obtained from compounds **1c** and **30a** according to the method of Example **1.**

¹H NMR (400 MHz, DMSO-*d₆*) δ 8.43-8.32 (m, 3H), 7.75-7.55 (m, 3H), 5.32 (brs, 1H), 5.55-5.30 (m, 8H), 1.69 (brs, 2H), 1.45-1.35 (m, 7H).

LCMS (ESI), [M+H]⁺ = 410.2.

### Example 31

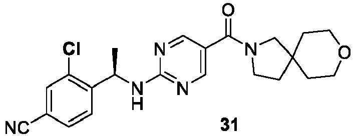

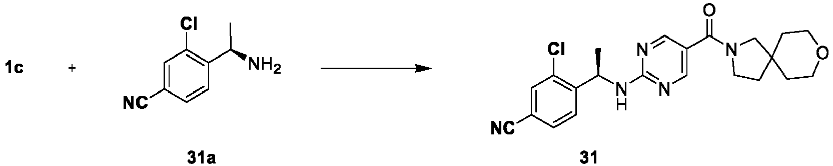

Compound **31** (52 mg) was obtained from compounds **1c** and **31a** according to the method of Example **1.**

¹H NMR (400 MHz, DMSO-*d₆*) δ 8.55-8.35 (m, 3H), 7.83 (s, 1H), 7.72 (brs, 2H), 5.50-5.39 (m, 1H), 3.61-3.40 (m, 8H), 1.81-1.71 (m, 2H), 1.56-1.37 (m, 7H).

LCMS (ESI), [M+H]⁺ = 425.1.

### Example 32

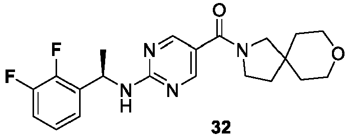

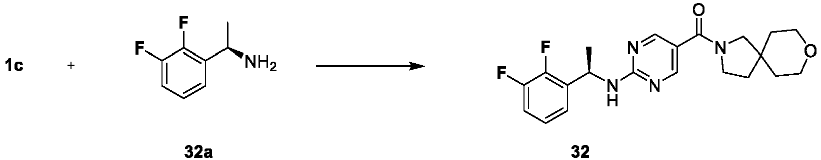

Compound **32** (52 mg) was obtained from compounds **1c** and **32a** according to the method of Example **1.**

¹H NMR (400 MHz, DMSO-*d₆*) δ 8.50 (brs, 2H), 8.35-8.33 (m, 1H), 7.28-7.16 (m, 3H), 5.43-5.40 (m, 1H), 3.61-3.40 (m, 8H), 1.78-1.76 (m, 2H), 1.56-1.37 (m, 7H).

LCMS (ESI), [M+H]⁺ = 403.2.

### Example 33

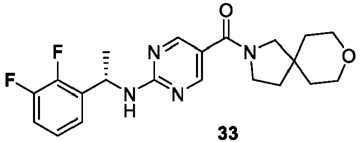

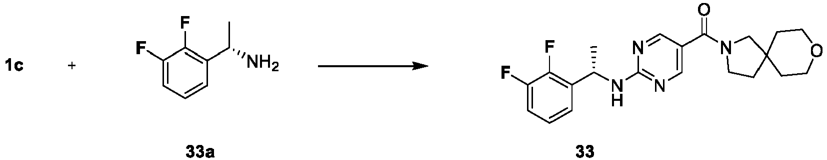

Compound **33** (28 mg) was obtained from compounds **1c** and **33a** according to the method of Example **1.**

¹H NMR (400 MHz, DMSO-*d₆*) δ 8.50 (brs, 2H), 8.35-8.33 (m, 1H), 7.28-7.16 (m, 3H), 5.43-5.40 (m, 1H), 3.65-3.48 (m, 8H), 1.78-1.76 (m, 2H), 1.56-1.37 (m, 7H).

LCMS (ESI), [M+H]⁺ = 403.2.

### Example 34

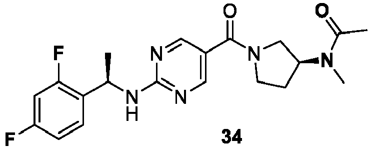

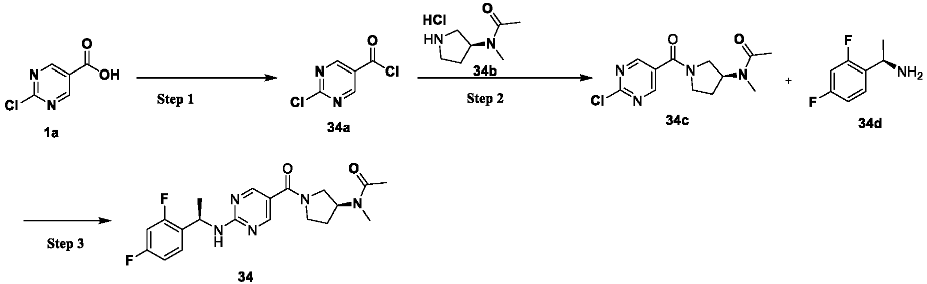

### Step 1

Compound 1a (2 g, 12.7 mmol) was dissolved in DCM (25 mL), and 1.6 mL of oxalyl chloride (2.42 g, 19 mmol) and 6 drops of DMF were added dropwise thereto under an ice bath. The ice bath was removed, and the reaction mixture was naturally warmed to room temperature and stirred continuously for 2 hours. After the reaction was completed, the solvent was removed under reduced pressure to obtain a crude product of **34a** (2.1 g).

### Step 2

Compound **34a** (2.1 g, 12 mmol) and compound **34b** (1.9 g, 11 mmol) were dissolved in acetonitrile (25 mL), and T₃P (20 mL, 60% in EA, 10 g, 31.25 mmol) was added dropwise thereto under an ice bath. After the dropwise addition was completed, the reaction mixture was naturally warmed to room temperature and stirred continuously for 2 hours. After the reaction was completed, the reaction mixture was quenched with water (50 mL), extracted with dichloromethane (20 mL×3), dried over anhydrous sodium sulfate, concentrated under vacuum, and purified by silica gel column (MeOH/DCM = 0-100) to obtain compound **34c** (600 mg).

### Step 3

Compound **34** (134 mg) was obtained from compounds **34c** and **34d** according to the method of Example **1.**

¹H NMR (400 MHz, DMSO-*d₆*) δ 8.51 (brs, 2H), 8.36-8.33 (m, 1H), 7.53-7.46 (m, 1H), 7.22-7.17 (m, 1H), 7.08-7.04 (m, 1H), 5.38 (brs, 1H), 5.03-4.48 (m, 1H), 3.68-3.35 (m, 4H), 2.86-2.71 (m, 3H), 2.15-1.91 (m, 5H), 1.46-1.44 (m, 3H).

LCMS (ESI), [M+H]⁺ = 404.1.

### Example 35

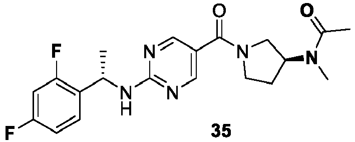

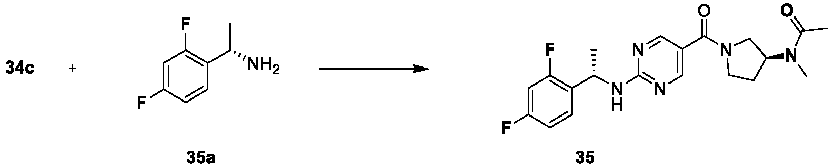

Compound **35** (134 mg) was obtained from compounds **34c** and **35a** according to the method of Example **1.**

¹H NMR (400 MHz, DMSO-*d₆*) δ 8.51 (brs, 2H), 8.36-8.33 (m, 1H), 7.53-7.46 (m, 1H), 7.22-7.17 (m, 1H), 7.08-7.04 (m, 1H), 5.38 (brs, 1H), 5.03-4.46 (m, 1H), 3.68-3.35 (m, 4H), 2.86-2.71 (m, 3H), 2.15-1.91 (m, 5H), 1.46-1.44 (m, 3H).

LCMS (ESI), [M+H]⁺ = 404.1.

### Example 36

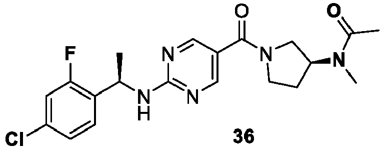

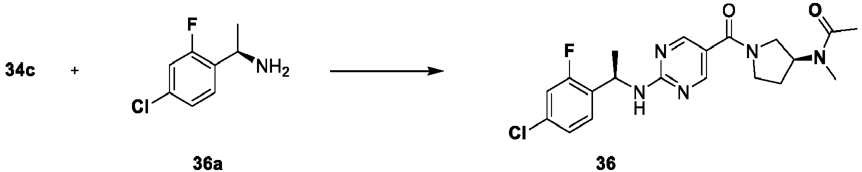

Compound **36** (12 mg) was obtained from compounds **34c** and **36a** according to the method of Example **1.**

¹H NMR (400 MHz, DMSO-*d₆*) δ 8.50 (brs, 2H), 8.36-8.33 (m, 1H), 7.47-7.25 (m, 3H), 5.36-5.32 (m, 1H), 4.94-4.47 (m, 1H), 3.69-3.40 (m, 4H), 2.86-2.71 (m, 3H), 2.03-1.83 (m, 5H), 1.46-1.41 (m, 3H).

LCMS (ESI), [M+H]⁺ = 420.1.

### Example 37

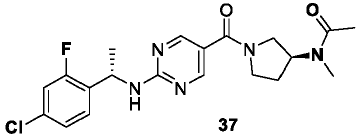

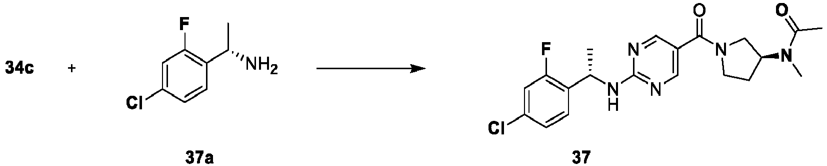

Compound **37** (12 mg) was obtained from compounds **34c** and **37a** according to the method of Example **1.**

¹H NMR (400 MHz, DMSO-*d₆*) δ 8.50 (brs, 2H), 8.36-8.32 (m, 1H), 7.47-7.24 (m, 3H), 5.36-5.32 (m, 1H), 4.94-4.47 (m, 1H), 3.69-3.40 (m, 4H), 2.86-2.71 (m, 3H), 2.03-1.83 (m, 5H), 1.46-1.41 (m, 3H).

LCMS (ESI), [M+H]⁺ = 420.1.

### Example 38

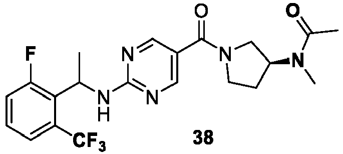

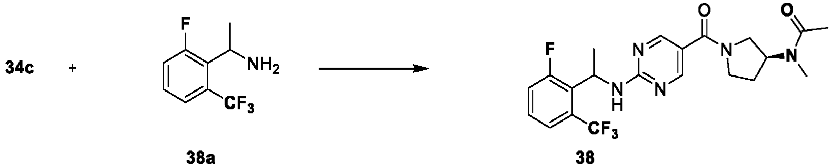

Compound 38 (41 mg) was obtained from compounds 34c and 38a according to the method of Example 1.

¹H NMR (400 MHz, DMSO-*d₆*) δ 8.47 (brs, 2H), 8.02-7.96 (m, 1H), 7.57-7.50 (m, 3H), 5.50-5.46 (m, 1H), 3.67-3.51 (m, 4H), 2.86-2.71 (m, 4H), 2.03-1.83 (m, 5H), 1.58-1.56 (m, 3H).

LCMS (ESI), [M+H]⁺ = 454.1.

### Example 39

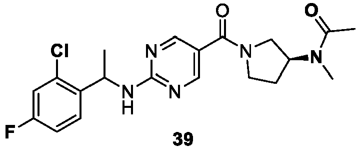

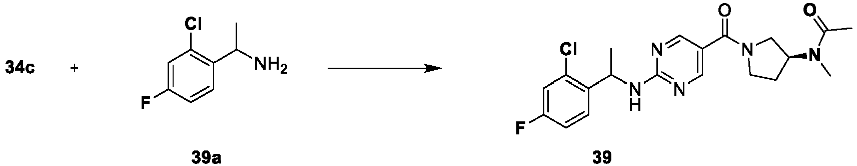

Compound 39 (100 mg) was obtained from compounds 34c and 39a according to the method of Example 1.

¹H NMR (400 MHz, DMSO-*d₆*) δ 8.49 (s, 2H), 8.40 (s, 1H), 7.57-7.52 (m, 1H), 7.41-7.55 (m, 1H), 7.23-7.18 (m, 1H), 5.45-5.36 (m, 1H), 3.66-3.59 (m, 4H), 2.85 (s, 1H), 2.71 (s, 1H), 2.05-1.97 (m, 6H), 1.45-1.40 (m, 3H), 1.23 (s, 1H).

LCMS (ESI), [M+H]⁺ = 420.1.

### Example 40

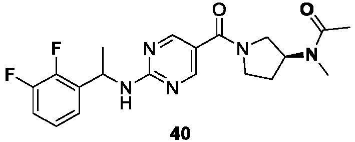

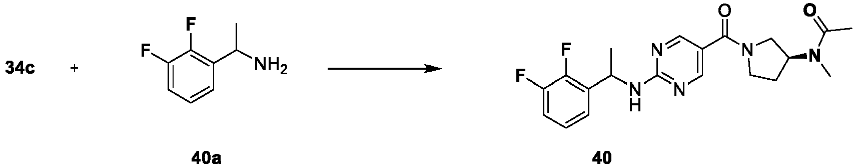

Compound 40 (100 mg) was obtained from compounds 34c and 40a according to the method of Example 1.

¹H NMR (400 MHz, DMSO-*d₆*) δ 8.51 (s, 2H), 8.39-8.35 (m, 1H), 7.28-7.25 (m, 2H), 7.20-7.12 (m, 1H), 5.43-5.40 (m, 1H), 3.64-3.46 (m, 4H), 2.85 (s, 1H), 2.71 (s, 1H), 2.05-1.94 (m, 6H), 1.50-1.45 (m, 3H), 1.24 (s, 1H).

LCMS (ESI), [M+H]⁺ = 404.2.

### Example 41

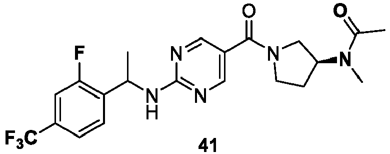

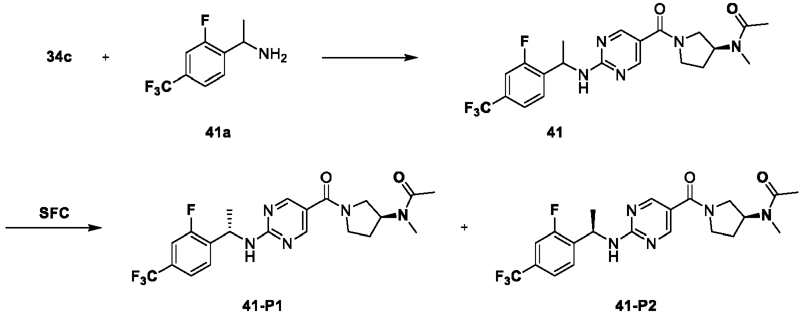

Compound 41 (1800 mg) was obtained from compounds 34c and 41a according to the method of Example 1.

¹H NMR (400 MHz, DMSO-*d₆*) δ 8.59-8.35 (m, 3H), 7.69-7.58 (m, 3H), 5.42-5.38 (m, 1H), 5.02-4.39 (m, 1H), 4.46 (brs, 1H), 3.69-3.44 (m, 4H), 3.00-2.85 (m, 3H), 2.03-1.85 (m, 4H), 1.48-1.42 (m, 3H).

LCMS (ESI), [M+H]⁺ = 454.2.

Two enantiomers 41-P1 and 41-P2 were obtained by chiral resolution of compound 41.

The chromatographic conditions are as follows:
Chromatographic column: CHIRALPAK IG (5 µm, 30 × 250 mm I.D)
Flow rate: 80 mL/min
Wavelength: 220 nm
Column temperature: 38°C
Mobile phase: A: CO₂, B: Methanol
Chiral synthesis of **41-P2**

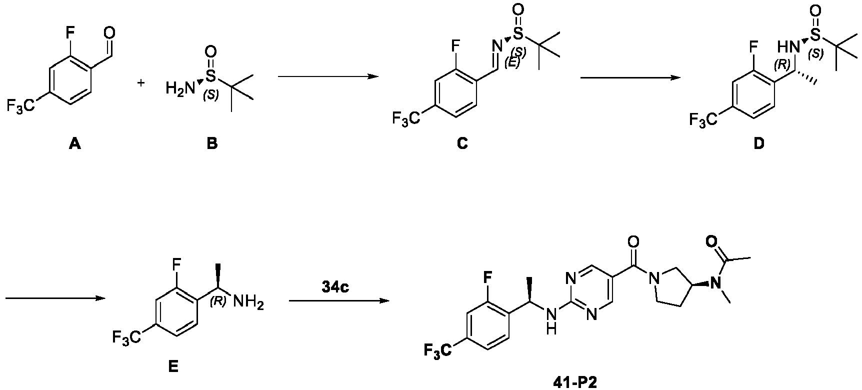

### Step 1

A solution of compound A (50 g, 0.26 mol), compound B (38 g, 0.31 mol) and tetraethyl titanate (120 g, 0.52 mol) in THF (500 mL) was heated to 40°C and stirred for 2 hours. After the reaction was completed, the reaction system was cooled down, added with 300 mL of water, stirred vigorously for 0.5 hours, filtered, then the filter cake was washed with water (100 mL × 1) and ethyl acetate (100 mL × 1) successively, and the filtrate was extracted with ethyl acetate (300 mL × 3). The organic phases were combined, dried and concentrated to obtain a crude product of C (72 g).

### Step 2

Compound C (72 g, 0.25 mol) was dissolved in 2-methyltetrahydrofuran (750 mL), and methylmagnesium bromide (3 M in 2-Me-THF, 420 mL) was slowly added dropwise thereto at -78°C. After the dropwise addition was completed, the reaction system was stirred continuously at -78°C for 2 hours. After the reaction was completed, the reaction mixture was quenched with saturated ammonium chloride aqueous solution (300 mL). Most of the 2-methyltetrahydrofuran was removed from the quenched mixture under reduced pressure, and the mixture was extracted with ethyl acetate (500 mL × 3). The organic phases were combined, dried and concentrated to obtain a crude product of **D** (78 g).

### Step 3

To a solution of compound D (40 g, 0.129 mol) in ethyl acetate (50 mL) was slowly added a solution of hydrochloric acid in ethyl acetate (3 M, 400 mL) at 0°C. After the dropwise addition was completed, the reaction mixture was stirred continuously at room temperature for 2 hours. After the reaction was completed, the reaction mixture was filtered to obtain a white solid. This white solid was dispersed into NaOH solution (4 N, 100 mL), extracted with ethyl acetate (300 mL × 2), dried and concentrated to obtain a crude product of E (16 g, Chiral HPLC: 78% ee). The crude product was subjected to chiral resolution to obtain target product E (9.6 g, Chiral HPLC: 100% ee).

### Step 4

Target product **41-P2** (9.7 g) was obtained from compound **E** (8.7 g, 0.038 mol) and compound **34c** (9.7 g, 0.034 mol) according to the synthesis method of Example 41.

¹H NMR (400 MHz, CD₃OD) δ 8.50 (s, 2H), 7.62-7.57 (m, 1H), 7.45-7.34 (m, 2H), 5.54-5.40 (m, 1H), 5.19-4.95 (m, 1H), 3.82-3.45 (m, 4H), 3.01-2.96 (m, 2H), 2.87-2.84 (m, 1H), 2.19-2.08 (m, 5H), 1.57-1.55 (m, 3H).

LCMS: [M+1] = 454.2.

Chiral HPLC conditions:
Mobile phase: 0.1% diethylamine + 70% n-hexane + 30% isopropylamine
Flow rate: 0.4 mL/min
Run time: 30 min
Column temperature: 30°C
Detection wavelength: 254 nm.

Through chiral HPLC analysis and comparison, the retention time (14.088 min) of the chiral compound 41-P2 obtained by the chiral synthesis method is the same as the retention time (14.018 min) of P2 obtained by SFC resolution of compound 41, so the absolute configuration of 41-P2 is determined as shown in the formula.

### Bioassay example

### 1. Vanin-1 recombinant enzyme activity inhibition assay

A certain mass of the compound was weighed precisely, and prepared with DMSO and reaction buffer (50 mM Tris base, 50 mM KCl, 1.6 mM cysteamine, 0.005% Brij 35, pH 8.0, prepared when using) to a maximum concentration of 10000 nM, then diluted in a 4-fold gradient, and prepared into 10 compound working solutions with different concentrations;
for the activity inhibition reaction of recombinant human Vanin-1 (Biorab, JN0618), 2.5 µL of compound working solution and 5 µL of recombinant human Vanin-1 protein were first mixed. The mixture was incubated at room temperature for 15 minutes, then added with 2.5 µL of Pantetheine 7-amino-4-trifluoromethylcoumarin substrate, such that the final concentration of recombinant human Vanin-1 was 62.5 pM and the final concentration of Pantetheine 7-amino-4-trifluoromethylcoumarin substrate was 45 µM in the 10 L reaction system. The reaction was carried out in a 384-well plate (PerkinElmer, 6007280) with DMSO at a final concentration of 1%. The excitation light was set at 405 nm and the emission light was set at 505 nm on the microplate reader, and kinetic reading was performed at 25°C for 1 hour. Raw data at the 30th minute were collected for data processing and analysis, then the concentration-effect curve was fitted with GraphPad Prism 8 software, and the IC₅₀ of the compound concentration was calculated. The data are shown in Table 1.

### 2. Human plasma Vanin-1 enzyme activity inhibition assay

For the activity inhibition reaction of human plasma, 2.5 µL of compound working solution and 5 µL of 100-fold diluted human plasma were first mixed. The mixture was incubated at room temperature for 15 minutes, then added with 2.5 µL of Pantetheine 7-amino-4-trifluoromethylcoumarin (internal synthesis) substrate, such that the final concentration of Pantetheine 7-amino-4-trifluoromethylcoumarin substrate was 25 µM in the 10 µL reaction system. The reaction was carried out in a 384-well plate (PerkinElmer, 6007280) with DMSO at a final concentration of 1%. The excitation light was set at 405 nm and the emission light was set at 505 nm on the microplate reader, and kinetic reading was performed at 25°C for 1 hour. Raw data at the 30th minute were collected for data processing and analysis, then the concentration-effect curve was fitted with GraphPad Prism 8 software, and the IC₅₀ of the compound concentration was calculated. The data are shown in Table 1 and Table 2.

**Table 1**

| Example | Vanin-1 IC₅₀ (nM) | Example | Vanin-1 IC₅₀ (nM) |
|---|---|---|---|
| Compound 1 | 13 | Compound 23 | 1.2 |
| Compound 2 | 411 | Compound 24 | 30.8 |
| Compound 3 | 323 | Compound 25 | 1.3 |
| Compound 4 | 1 | Compound 26 | 14.5 |
| Compound 5 | 65 | Compound 27 | 0.4 |
| Compound 6 | 6 | Compound 28 | 0.8 |
| Compound 7 | 46 | Compound 29 | 0.4 |
| Compound 8 | 18 | Compound 30 | 2.1 |
| Compound 9 | 36 | Compound 32 | 6.8 |
| Compound 10 | 8 | Compound 33 | 1.1 |
| Compound 11 | 40 | Compound 34 | 10.7 |
| Compound 12 | 17 | Compound 35 | 0.5 |
| Compound 13 | 74 | Compound 36 | 23.4 |
| Compound 14 | 38 | Compound 37 | 0.4 |
| Compound 15 | 0.4 | Compound 38 | 0.1 |
| Compound 16 | 211 | Compound 39 | 1.8 |
| Compound 17 | 82 | Compound 41 | 0.2 |
| Compound 18 | 1.4 | Compound 41-P2 | 0.3 |
| Compound 20 | 2.3 | Compound A | 11.25 |
| Compound 22 | 39.1 | | |

**Table 2**

| Example | hPlasma IC₅₀ (nM) |
|---|---|
| Compound 4 | 0.5 |
| Compound 6 | 1.6 |
| Compound 10 | 5.7 |
| Compound 12 | 7.9 |
| Compound 14 | 28 |
| Compound 15 | 0.1 |
| Compound 18 | 0.5 |
| Compound A | 6.6 |

Compound A is 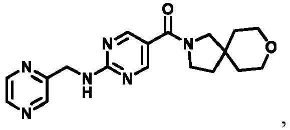 which was prepared with reference to the method of Example 142 in CN109476645A.

### 3. Drug-drug interaction (CYP enzyme inhibitory activity IC₅₀ assay)

In this experiment, the cocktail method was used to study the inhibitory effect of the compound to be tested on the activities of 1A2, 2B6, 2C8, 2C19, 2C9, 2D6 and 3A4 subtypes of CYP enzyme, so as to measure the IC₅₀ values for the inhibitory effect of the compound to be tested on the activities of the above several CYP enzyme subtypes. The CYP enzyme probe substrates used in the experiment were: Phenacetin (1A2), Bupropion (2B6), Amodiaquine (2C8), Mephenytoin (2C19), Diclofenac (2C9), Dextromethorphan (2D6) and Testosterone (3A4/5). The final concentration of microsomes in the experimental system was 0.1 mg/mL. PBS Buffer was 50 mM K₂HPO₄ buffer. The concentrations of the compound to be tested were 50 µM, 12.5 µM, 3.125 µM, 0.781 µM, 0.195 µM, and 0.0488 µM, respectively. The corresponding probe substrates and microsomes were added into PBS, mixed well and dispensed into each reaction system, then control compound/compound to be tested/DMSO solution was added into the corresponding reaction systems respectively. The reaction system was mixed well, pre-incubated in a water bath at 37°C for 5 minutes, added with 10 mM NADPH solution and mixed well, and reacted in a water bath at 37°C for 10 minutes. After the reaction was completed, an internal standard acetonitrile solution was added to terminate the reaction. Centrifugation was performed at 4000 rpm, and the supernatant solution was taken and mixed well with an equal volume of pure water. The amount of reaction product from each probe substrate was analyzed by LC-MS/MS (AB Triple Quard 5500) respectively, and the measured amount of reaction product was used for IC₅₀ calculation and graphing with GraphPad Prism 5. The measured data for some of the compounds of the present disclosure are shown in Table 3.

**Table 3**

| Compound No. | IC₅₀ (µM) | | | | |
|---|---|---|---|---|---|
| | 1A2 | 2C9 | 2C 19 | 2D6 | 3A4 |
| 41-P2 | >50 | 45.6 | >50 | >50 | >50 |

It can be seen from Table 3 that compound 41-P2 has basically no inhibitory effect on the tested CYP enzymes.

### 4. In vitro liver microsome stability assay

In this experiment, the final concentration of microsomes in the experimental system was 0.5 mg/mL. PBS Buffer was 50 mM K₂HPO₄ buffer. The concentration of the compound to be tested was 1 µM. The microsomes were added into PBS, mixed well and dispensed into each reaction system, and control compound/compound to be tested was added into the corresponding reaction systems respectively. The reaction system was mixed well, pre-incubated in a water bath at 37°C for 5 minutes, added with 20 mM NADPH solution and mixed well, and the reaction was initiated in a water bath at 37°C. At the reaction time points of 0 min, 10 min, 30 min, 60 min and 90 min, respectively, 30 µL of the reaction sample was taken from each reaction system, and 300 µL of the internal standard acetonitrile solution was added immediately to terminate the reaction. After centrifugation at 4000 rpm, the supernatant solution was taken and mixed well with an equal volume of pure water. The amount of compound in the sample at each time point was detected by LC-MS/MS (AB Triple Quard 5500) respectively, and the parameters such as T1/2, *In vitro* CLint (µL/min/mg protein) and CLhepatic (mL/min/kg) were calculated using Microsoft Excel 2010. The measured data for some of the compounds of the present disclosure are shown in Table 4.

**Table 4**

| Compound No. | T_{1/2} (min) | | |
|---|---|---|---|
| | Human | Mouse | Rat |
| 41-P2 | >279.6 | 98.8 | 198.4 |

It can be seen from Table 4 that the liver microsomes of compound 41-P2 have remarkable stability.

### 5. In vivo pharmacokinetic evaluation in mice

The vehicle used in the experiment was: DMSO: Solutol: PBS = 5%:25%:70% (v/v/v). Preparation method: The required compound was weighed accurately, added with a certain volume of DMSO in a certain proportion, vortex-mixed until completely dissolved, then added successively with Solutol and PBS according to the above proportion, and mixed well. The vehicles used in the intravenous (iv) administration group and oral (po) administration group were the same vehicles in the experiment. The intravenous dose was 2 mpk and the oral dose was 5 mpk. Experimental blood collection time points: IV group: 0.083, 0.25, 0.5, 1, 2, 4, 7, 24 h. PO group: 200 µL of whole blood was collected from jugular vein at each time point of 0.25, 0.5, 1, 2, 4, 7 and 24 h, anticoagulated with EDTA-K2, and immediately centrifuged at 4000 rpm for 5 minutes at 4°C. The supernatant was taken and the samples were frozen and stored in a refrigerator at -80°C. Processing of plasma samples: the sample was precipitated with ACN/MeOH (1: 1, v/v) precipitant containing internal standard, then centrifuged at 14000 rpm for 5 minutes. The supernatant was taken into LC-MS/MS (AB Triple Quard 5500) for analysis to obtain plasma concentrations, and parameters were calculated by the non-compartmental model of Winnolin 8.1 version. The results are shown in Table 5.

**Table 5**

| Compound | Mode of administration | Peak concentration Cₘₐₓ (ng/mL) | Half-life T_{1/2} (hr) | Area under the curve AUC₀₋₇ₕ (hr×ng/mL) | Distributed solvent Vd (L/kg) | Clearance rate CL (mL/hr/kg) | Bioavailability F% |
|---|---|---|---|---|---|---|---|
| Compound 41-P2 | Intravenous injection | 2145.0 | 2.80 | 4959.1 | 1.26 | 6.73 | -- |
| | Intragastric administration | 1536.0 | 5.60 | 10656.5 | -- | -- | 86 |

| | | | | | | | |
|---|---|---|---|---|---|---|---|
| Note: "-" in the table means not applicable. | | | | | | | |

As can be seen from Table 5, compound 41-P2 had an exposure of 10656.5 hr×ng/mL at the given dose and exhibited high bioavailability of 86% in mice, showing that the compound of the present disclosure has excellent pharmacokinetic properties in mice. Although the specific embodiments of the present disclosure have been described above, it should be understood by those skilled in the art that these are merely illustrative examples and that a variety of changes or modifications can be made to these embodiments without departing from the principles and substance of the present disclosure. Therefore, the scope of protection of the present disclosure is limited by the appended claims.

## Claims

1. A pyrimidine carboxamide compound represented by formula I, or a tautomer, mesomer, racemate, enantiomer, diastereomer thereof, or a mixture form thereof, or a pharmaceutically acceptable salt thereof; 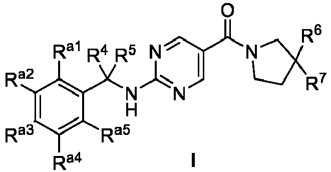
wherein, R^{a1}, R^{a3} and R^{a5} are independently H, halogen, CN, C₁-C₆ alkyl, C₁-C₆ alkyl-O-, C₁-C₆ alkyl substituted by one or more than one R^{1a}, or C₁-C₆ alkyl-O- substituted by one or more than one R^{1b};
R^{a2} and R^{a4} are independently H, halogen;
R⁴ and R⁵ are independently H, C₁-C₆ alkyl, C₁-C₆ alkyl substituted by one or more than one halogen; alternatively, R⁴ and R⁵ form 3- to 7-membered cycloalkyl together with the carbon to which they are bonded;
R⁶ and R⁷ are independently H, halogen, C₁-C₆ alkyl, C₁-C₆ alkyl substituted by one or more than one R^{1c}, -N(R^{2a}R^{2b}) or -N(R^{3a})-(C=O)-R^{3b};
alternatively, R⁶ and R⁷ form a ring B together with the carbon to which they are bonded; the ring B is 4- to 7-membered cycloalkyl, 4- to 7-membered heterocycloalkyl, 4- to 7-membered cycloalkyl substituted by one or more than one R^{1d}, or 4- to 7-membered heterocycloalkyl substituted by one or more than one R^{1e}; the heteroatom of the 4- to 7-membered heterocycloalkyl in the 4- to 7-membered heterocycloalkyl and the 4- to 7-membered heterocycloalkyl substituted by one or more than one R^{1e} is N, O or S, and the number of heteroatom is 1 or 2;
R^{1a}, R^{1b}, R^{1c}, R^{1d} and R^{1e} are independently halogen, C₁-C₄ alkyl or C₁-C₄ alkyl substituted by one or more than one halogen;
R^{2a}, R^{2b}, R^{3a} and R^{3b} are independently H or C₁-C₄ alkyl.

2. The pyrimidine carboxamide compound represented by formula I, or the tautomer, mesomer, racemate, enantiomer, diastereomer thereof, or the mixture form thereof, or the pharmaceutically acceptable salt thereof according to claim 1, wherein,
R^{a1}, R^{a3} and R^{a5} are independently H, halogen, CN, C₁-C₆ alkyl-O-, C₁-C₆ alkyl substituted by one or more than one R^{1a}, or C₁-C₆ alkyl-O- substituted by one or more than one R^{1b};
and/or, 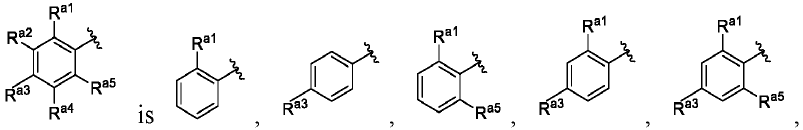 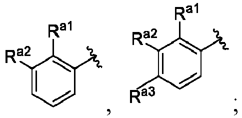
and/or, R^{1a} and R^{1b} are independently halogen;
and/or, the number of R^{1a} and R^{1b} is 1, 2, 3, 4 or 5;
and/or, 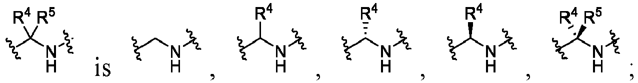 R⁴ and R⁵ are independently C₁-C₆ alkyl; alternatively, R⁴ and R⁵ form 3- to 7-membered cycloalkyl together with the carbon to which they are bonded;
and/or, R⁶ and R⁷ are independently H, N(R^{2a}R^{2b}) or -N(R^{3a})-(C=O)-R^{3b}; alternatively, R⁶ and R⁷ form the ring B together with the carbon to which they are bonded;
and/or, the ring B is 4- to 7-membered heterocycloalkyl;
and/or, R^{2a} and R^{2b} are independently C₁-C₄ alkyl;
and/or, R^{3a} and R^{3b} are independently C₁-C₄ alkyl.

3. The pyrimidine carboxamide compound represented by formula I, or the tautomer, mesomer, racemate, enantiomer, diastereomer thereof, or the mixture form thereof, or the pharmaceutically acceptable salt thereof according to claim 2, wherein,
R^{a1}, R^{a3} and R^{a5} are independently H, halogen, C₁-C₆ alkyl-O-, C₁-C₆ alkyl substituted by one or more than one R^{1a}, or C₁-C₆ alkyl-O- substituted by one or more than one R^{1b};
and/or, 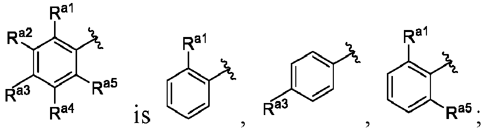
and/or, the number of R^{1a} and R^{1b} is 2 or 3;
and/or, 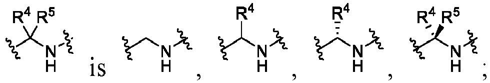 R⁴ and R⁵ are independently C₁-C₆ alkyl; alternatively, R⁴ and R⁵ form 3- to 7-membered cycloalkyl together with the carbon to which they are bonded;
and/or, R⁶ and R⁷ form the ring B together with the carbon to which they are bonded;
and/or, when R^{a1}, R^{a2}, R^{a3}, R^{a4} and R^{a5} are independently halogen, the halogen is fluorine, chlorine or bromine;
and/or, when R^{a1}, R^{a3} and R^{a5} are independently C₁-C₆ alkyl, C₁-C₆ alkyl-O-, C₁-C₆ alkyl substituted by one or more than one R^{1a}, or C₁-C₆ alkyl-O- substituted by one or more than one R^{1b}, the C₁-C₆ alkyl in the C₁-C₆ alkyl, C₁-C₆ alkyl-O-, C₁-C₆ alkyl substituted by one or more than one R^{1a} and C₁-C₆ alkyl-O- substituted by one or more than one R^{1b} is independently methyl, ethyl, n-propyl, isopropyl, n-butyl, isobutyl, sec-butyl or tert-butyl;
and/or, when R⁴ and R⁵ are independently C₁-C₆ alkyl, or C₁-C₆ alkyl substituted by one or more than one halogen, the C₁-C₆ alkyl in the C₁-C₆ alkyl and the C₁-C₆ alkyl substituted by one or more than one halogen is independently methyl, ethyl, *n*-propyl, isopropyl, *n*-butyl, isobutyl, *sec*-butyl or *tert*-butyl;
and/or, when R⁴ and R⁵ are independently C₁-C₆ alkyl substituted by one or more than one halogen, the halogen is fluorine, chlorine or bromine;
and/or, when R⁴ and R⁵ form 3- to 7-membered cycloalkyl together with the carbon to which they are bonded, the 3- to 7-membered cycloalkyl is independently cyclobutyl, cyclopentyl, cyclohexyl or cycloheptyl;
and/or, when R⁶ and R⁷ are independently halogen, the halogen is fluorine, chlorine or bromine;
and/or, when R⁶ and R⁷ are independently C₁-C₆ alkyl, or C₁-C₆ alkyl substituted by one or more than one R^{1c}, the C₁-C₆ alkyl in the C₁-C₆ alkyl and the C₁-C₆ alkyl substituted by one or more than one R^{1c} is independently methyl, ethyl, n-propyl, isopropyl, n-butyl, isobutyl, *sec-*butyl or *tert*-butyl;
and/or, when the ring B is 4- to 7-membered cycloalkyl, or 4- to 7-membered cycloalkyl substituted by one or more than one R^{1d}, the 4- to 7-membered cycloalkyl in the 4- to 7-membered cycloalkyl and the 4- to 7-membered cycloalkyl substituted by one or more than one R^{1d} is independently cyclobutyl, cyclopentyl, cyclohexyl or cycloheptyl;
and/or, and when the ring B is 4- to 7-membered heterocycloalkyl, or 4- to 7-membered heterocycloalkyl substituted by one or more than one R^{1e}, the heteroatom of the 4- to 7-membered heterocycloalkyl in the 4- to 7-membered heterocycloalkyl and the 4- to 7-membered heterocycloalkyl substituted by one or more than one R^{1e} is N or O, and the number of the heteroatom is 1;
and/or, when R^{1a}, R^{1b}, R^{1c}, R^{1d} and R^{1e} are independently halogen, or C₁-C₄ alkyl substituted by one or more than one halogen, the halogen in the halogen and the C₁-C₄ alkyl substituted by one or more than one halogen is independently fluorine, chlorine or bromine;
and/or, when R^{1a}, R^{1b}, R^{1c}, R^{1d} and R^{1e} are independently C₁-C₄ alkyl, or C₁-C₄ alkyl substituted by one or more than one halogen, the C₁-C₄ alkyl in the C₁-C₄ alkyl and the C₁-C₄ alkyl substituted by one or more than one halogen is independently methyl, ethyl, n-propyl, isopropyl, n-butyl, isobutyl, sec-butyl or tert-butyl;
and/or, when R^{2a}, R^{2b}, R^{3a} and R^{3b} are independently C₁-C₄ alkyl, the C₁-C₄ alkyl is independently methyl, ethyl, n-propyl, isopropyl, n-butyl, isobutyl, sec-butyl or tert-butyl.

4. The pyrimidine carboxamide compound represented by formula I, or the tautomer, mesomer, racemate, enantiomer, diastereomer thereof, or the mixture form thereof, or the pharmaceutically acceptable salt thereof according to claim 3, wherein,
R^{a1}, R^{a3} and R^{a5} are independently H, halogen, or C₁-C₆ alkyl substituted by one or more than one R^{1a};
and/or, when R^{a1}, R^{a2}, R^{a3}, R^{a4} and R^{a5} are independently halogen, the halogen is fluorine or chlorine;
and/or, when R^{a1}, R^{a3} and R^{a5} are independently C₁-C₆ alkyl, C₁-C₆ alkyl-O-, C₁-C₆ alkyl substituted by one or more than one R^{1a}, or C₁-C₆ alkyl-O- substituted by one or more than one R^{1b}, the C₁-C₆ alkyl in the C₁-C₆ alkyl, C₁-C₆ alkyl-O-, C₁-C₆ alkyl substituted by one or more than one R^{1a} and C₁-C₆ alkyl-O- substituted by one or more than one R^{1b} is independently methyl or isopropyl;
and/or, when R⁴ and R⁵ are independently C₁-C₆ alkyl, C₁-C₆ alkyl substituted by one or more than one halogen, the C₁-C₆ alkyl in the C₁-C₆ alkyl and the C₁-C₆ alkyl substituted by one or more than one halogen is independently methyl;
and/or, when R⁴ and R⁵ are independently C₁-C₆ alkyl substituted by one or more than one halogen, the halogen is fluorine or chlorine;
and/or, when R⁴ and R⁵ form 3- to 7-membered cycloalkyl together with the carbon to which they are bonded, the 3- to 7-membered cycloalkyl is independently cyclopropyl;
and/or, 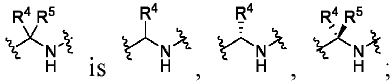 R⁴ and R⁵ are independently C₁-C₆ alkyl; alternatively, R⁴ and R⁵ form 3- to 7-membered cycloalkyl together with the carbon to which they are bonded;
and/or, R⁶ and R⁷ form the ring B together with the carbon to which they are bonded; the ring B is 4- to 7-membered heterocycloalkyl;
and/or, when the ring B is 4- to 7-membered heterocycloalkyl, or 4- to 7-membered heterocycloalkyl substituted by one or more than one R^{1e}, the 4- to 7-membered heterocycloalkyl in the 4- to 7-membered heterocycloalkyl and the 4- to 7-membered heterocycloalkyl substituted by one or more than one R^{1e} is tetrahydropyranyl;
and/or, when R^{1a}, R^{1b}, R^{1c}, R^{1d} and R^{1e} are independently halogen, or C₁-C₄ alkyl substituted by one or more than one halogen, the halogen in the halogen and the C₁-C₄ alkyl substituted by one or more than one halogen is independently fluorine or chlorine;
and/or, when R^{1a}, R^{1b}, R^{1c}, R^{1d} and R^{1e} are independently C₁-C₄ alkyl, or C₁-C₄ alkyl substituted by one or more than one halogen, the C₁-C₄ alkyl in the C₁-C₄ alkyl and the C₁-C₄ alkyl substituted by one or more than one halogen is independently methyl;
and/or, when R^{a1}, R^{a3} and R^{a5} are independently C₁-C₆ alkyl substituted by one or more than one R^{1a} or C₁-C₆ alkyl-O- substituted by one or more than one R^{1b}, the R^{a1}, R^{a3} and R^{a5} are independently trifluoromethyl, trifluoromethyl-O-;
and/or, when R^{2a}, R^{2b}, R^{3a} and R^{3b} are independently C₁-C₄ alkyl, the C₁-C₄ alkyl is independently methyl.

5. The pyrimidine carboxamide compound represented by formula I, or the tautomer, mesomer, racemate, enantiomer, diastereomer thereof, or the mixture form thereof, or the pharmaceutically acceptable salt thereof according to claim 1, wherein, 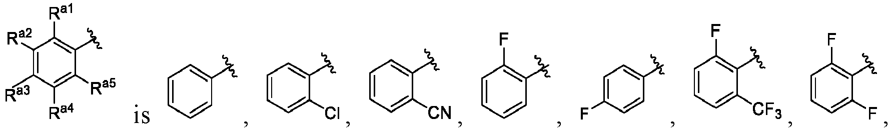 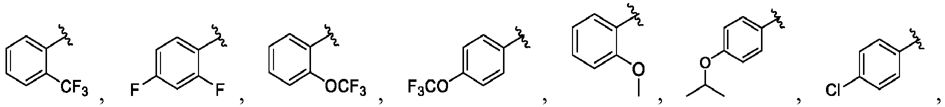 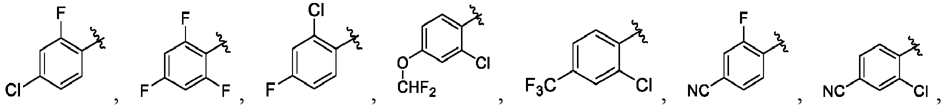 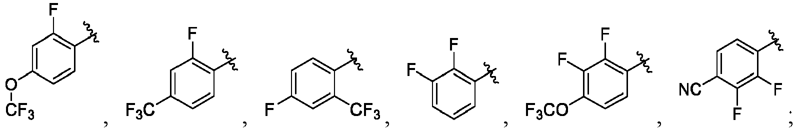 and/or, 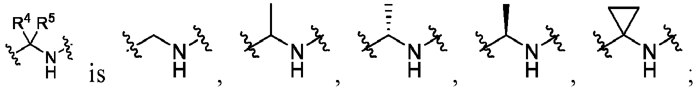 and/or,

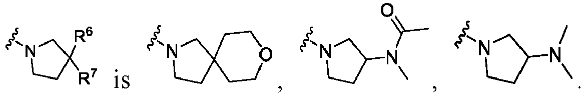

6. The pyrimidine carboxamide compound represented by formula I, or the tautomer, mesomer, racemate, enantiomer, diastereomer thereof, or the mixture form thereof, or the pharmaceutically acceptable salt thereof according to claim 1, wherein, the pyrimidine carboxamide compound represented by formula I is adapted to scheme 1, scheme 2, scheme 3, scheme 4;
scheme 1:
R^{a1}, R^{a3} and R^{a5} are independently H, halogen, CN, C₁-C₆ alkyl-O-, C₁-C₆ alkyl substituted by one or more than one R^{1a}, or C₁-C₆ alkyl-O- substituted by one or more than one R^{1b};
R^{a2} and R^{a4} are independently H, halogen;
R⁴ and R⁵ are independently H, C₁-C₆ alkyl; alternatively, R⁴ and R⁵ form 3- to 7-membered cycloalkyl together with the carbon to which they are bonded;
R⁶ and R⁷ are independently H, -N(R^{2a}R^{2b}) or -N(R^{3a})-(C=O)-R^{3b};
alternatively, R⁶ and R⁷ form the ring B together with the carbon to which they are bonded; the ring B is 4- to 7-membered heterocycloalkyl;
R^{1a} is independently halogen;
R^{2a}, R^{2b}, R^{3a} and R^{3b} are independently H or C₁-C₄ alkyl;
scheme 2:
R^{a1}, R^{a3} and R^{a5} are independently H, halogen, CN, C₁-C₆ alkyl-O-, C₁-C₆ alkyl substituted by one or more than one R^{1a}, or C₁-C₆ alkyl-O- substituted by one or more than one R^{1b};
R^{a2} and R^{a4} are independently H, halogen;
R⁴ and R⁵ are independently H, C₁-C₆ alkyl; alternatively, R⁴ and R⁵ form 3- to 7-membered cycloalkyl together with the carbon to which they are bonded;
R⁶ and R⁷ form the ring B together with the carbon to which they are bonded; the ring B is 4- to 7-membered heterocycloalkyl;
R^{1a} is independently halogen;
scheme 3:
R^{a1}, R^{a2}, R^{a3}, R^{a4} and R^{a5} are independently H or halogen; 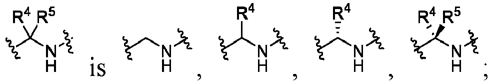 R⁴ and R⁵ are independently C₁-C₆ alkyl;
alternatively, R⁴ and R⁵ form 3- to 7-membered cycloalkyl together with the carbon to which they are bonded;
R⁶ and R⁷ form the ring B together with the carbon to which they are bonded; the ring B is 4- to 7-membered heterocycloalkyl;
scheme 4:
R^{a1}, R^{a2}, R^{a3}, R^{a4} and R^{a5} are independently H or halogen; 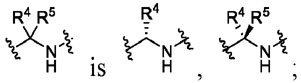 R⁴ and R⁵ are independently C₁-C₆ alkyl; alternatively, R⁴ and R⁵ form 3- to 7-membered cycloalkyl together with the carbon to which they are bonded;
R⁶ and R⁷ form the ring B together with the carbon to which they are bonded; the ring B is 4- to 7-membered heterocycloalkyl.

7. The pyrimidine carboxamide compound represented by formula I, or the tautomer, mesomer, racemate, enantiomer, diastereomer thereof, or the mixture form thereof, or the pharmaceutically acceptable salt thereof according to claim 1, wherein,
the pyrimidine carboxamide compound represented by formula I is selected from any one of the following structures:

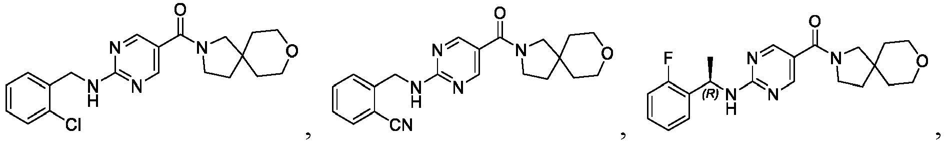

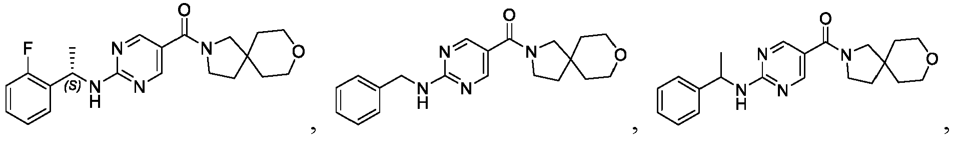

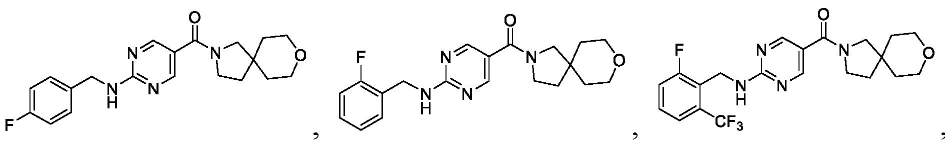

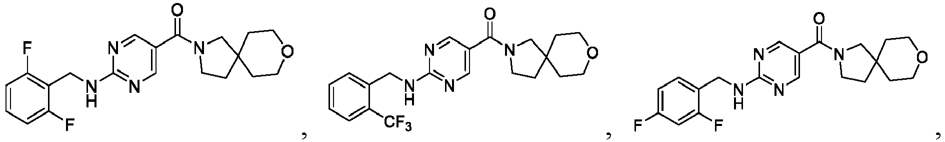

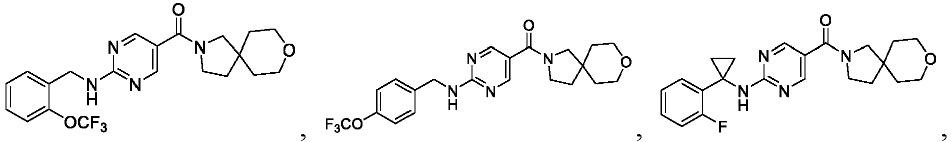

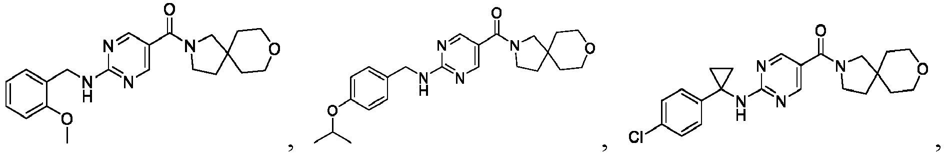

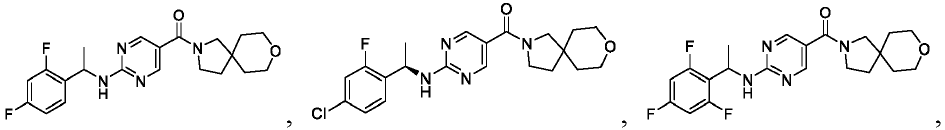

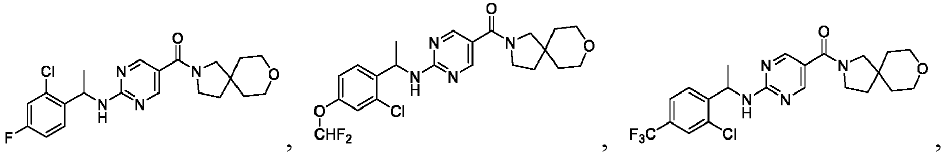

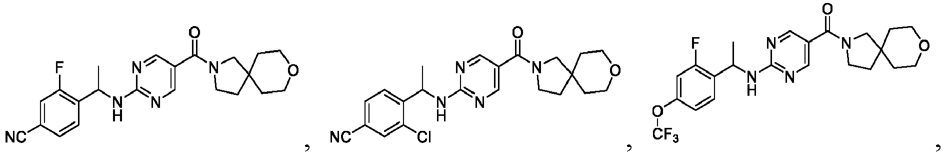

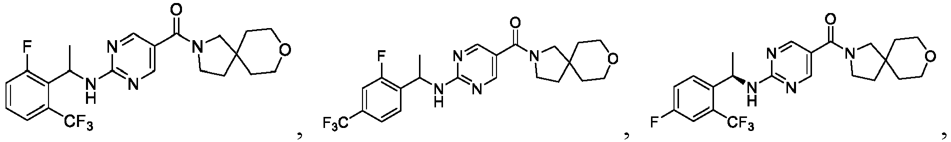

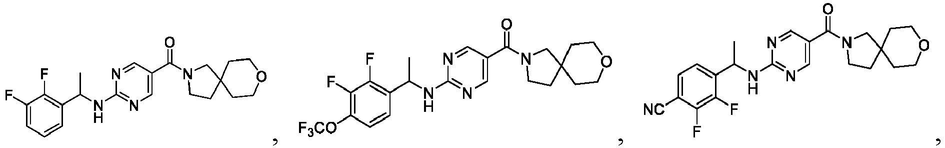

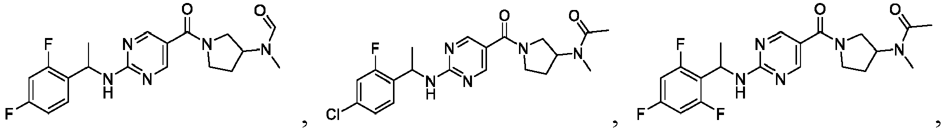

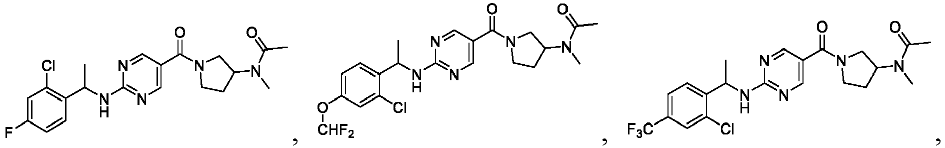

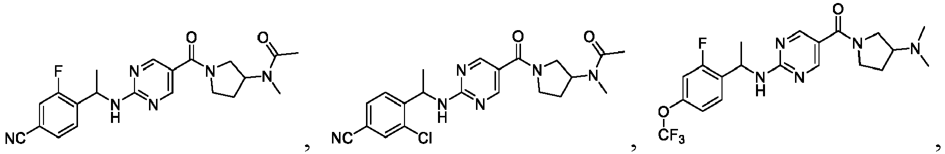

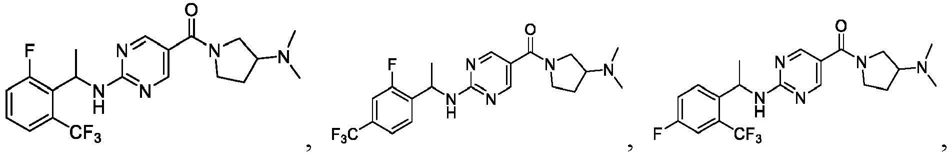

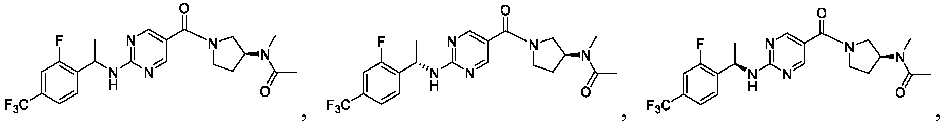

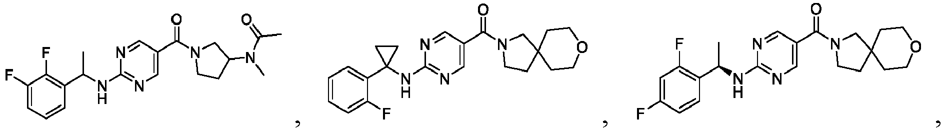

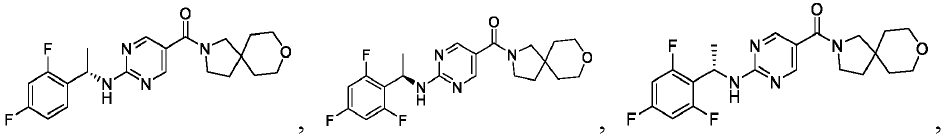

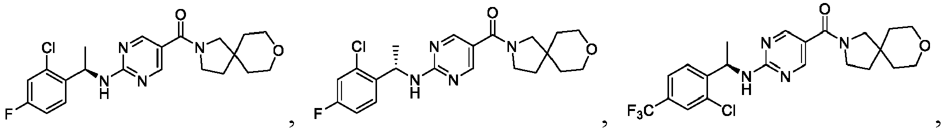

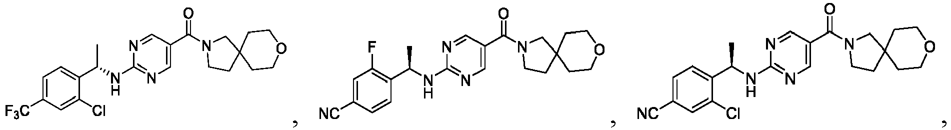

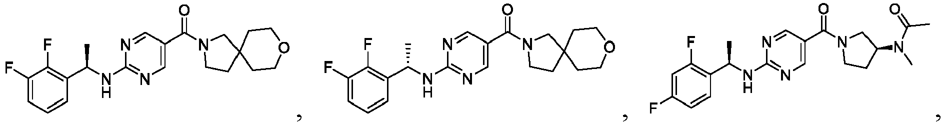

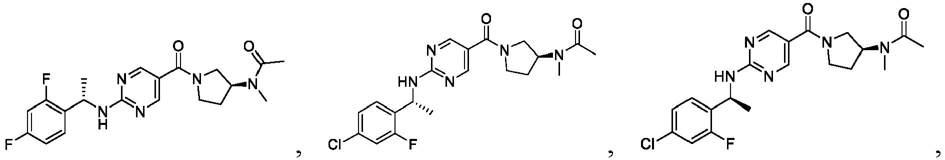

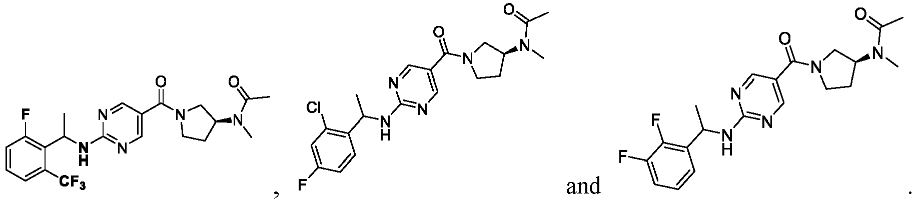

8. A pharmaceutical composition, comprising the pyrimidine carboxamide compound represented by formula I, or the tautomer, mesomer, racemate, enantiomer, diastereomer thereof, or the mixture form thereof, or the pharmaceutically acceptable salt thereof according to any one of claims 1 to 7, and a pharmaceutical excipient.

9. A use of the pyrimidine carboxamide compound represented by formula I, or the tautomer, mesomer, racemate, enantiomer, diastereomer thereof, or the mixture form thereof, or the pharmaceutically acceptable salt thereof according to any one of claims 1 to 7 in the manufacture of a Vanin-1 inhibitor.

10. A use of the pyrimidine carboxamide compound represented by formula I, or the tautomer, mesomer, racemate, enantiomer, diastereomer thereof, or the mixture form thereof, or the pharmaceutically acceptable salt thereof according to any one of claims 1 to 7 in the manufacture of a drug;
the drug is used to prevent and/or treat diseases related to Vanin-1, or, the drug is used to prevent and/or treat one or more of autoimmune diseases, inflammatory diseases, allergic diseases, metabolic diseases, infection-based diseases, fibrotic diseases, cardiovascular diseases, respiratory diseases, renal diseases, dermatological diseases, liver diseases, gastrointestinal diseases, oral diseases and hematopoietic diseases; for example, Crohn's disease, ulcerative colitis, inflammatory bowel disease and gastritis;
the diseases related to Vanin-1 may comprise one or more of autoimmune diseases, inflammatory diseases, allergic diseases, metabolic diseases, infection-based diseases, fibrotic diseases, cardiovascular diseases, respiratory diseases, renal diseases, dermatological diseases, liver diseases, gastrointestinal diseases, oral diseases and hematopoietic diseases; for example, Crohn's disease, ulcerative colitis, inflammatory bowel disease and gastritis.
